# EUROPEAN PATENT SPECIFICATION

(11) **EP 2 398 468 B1**
(45) Date of publication and mention of the grant of the patent: **30.11.2016**
(21) Application number: 10704517.1
(22) Date of filing: 17.02.2010
(51) Int. Cl.: A61K 9/20, A61K 9/28, A61K 31/4365, A61P 7/02

(54) **PHARMACEUTICAL COMPOSITIONS COMPRISING PRASUGREL BASE OR ITS PHARMACEUTICALLY ACCEPTABLE ACID ADDITION SALTS AND PROCESSES FOR THEIR PREPARATION**
PHARMAZEUTISCHE ZUSAMMENSETZUNGEN MIT EINER PRASUGREL-BASIS ODER IHREN PHARMAZEUTISCH ANNEHMBAREN SÄUREADDITIONSSALZEN UND HERSTELLUNGSVERFAHREN DAFÜR
COMPOSITIONS PHARMACEUTIQUES COMPRENANT DU PRASUGREL OU SES SELS D'ADDITION D'ACIDE PHARMACEUTIQUEMENT ACCEPTABLES, ET LEURS PROCÉDÉS DE PRÉPARATION

(30) Priority: 17.02.2009 SI 200900040; 02.06.2009 SI 200900155; 09.07.2009 SI 200900186; 15.07.2009 SI 200900194; 20.10.2009 SI 200900309; 28.12.2009 SI 200900396; 14.01.2010 SI 201000006
(43) Date of publication of application: 28.12.2011
(73) Proprietor: KRKA, D.D., Novo Mesto, 8501 Novo mesto (SI)
(72) Inventor: KUKEC, Simon, 8295 Trzisce (SI); STUKELJ, Mitja, 8222 Otocec (SI); VRECER, Franc, 8351 Straza (SI); TROST, Sabina, 1330 Kocevje (SI); MEZNAR, Klavdija, 8000 Novo mesto (SI); KLJAJIC, Alen, 3000 Celje (SI)
(74) Representative: Andrae | Westendorp Patentanwälte Partnerschaft
(86) International application number: PCT/EP2010/000997
(87) International publication number: WO 2010/094471

(56) References cited:
- EP-A1- 2 100 606
- EP-A1- 2 100 609
- WO-A2-2004/098713
- WO-A2-2006/135605
- WO-A2-2008/060934

## Description

The subject of the invention includes a pharmaceutical composition comprising micronized prasugrel or its pharmaceutically acceptable salts as the active pharmaceutical ingredient, prepared in the absence of water with solvent free technological methods and characterized in that it does not contain lactose.

### BACKGROUND OF THE INVENTION

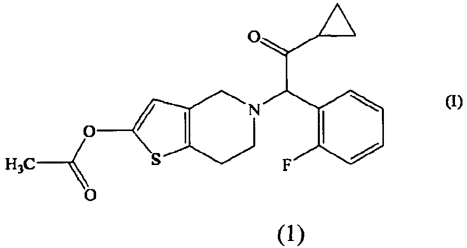

Prasugrel (1) is a next generation thienopyridine currently undergoing clinical development for the treatment of thrombosis and/or related diseases including as an adjunct to percutaneous coronary intervention procedures.

EP 542411 discloses and claims tetrahydrothienopyridine derivatives including 2-acetoxy-5-([alpha]-cyclopropylcarbonyl-2-fluorobenzyl)-4,5,6,7-tetrahydrothieno[3,2-c]pyridine (prasugrel).

EP1298132 discloses and claims the hydrochloric acid and EP1728794 describes the maleic acid salts of 2-acetoxy-5-([alpha]-cyclopropylcarbonyl-2-fluorobenzyl)-4,5,6,7-tetrahydrothieno[3,2-c]pyridine. The HCl and maleate salt forms are claimed to provide unexpected and unobvious improvements in their efficacy and stability profiles compared to other salts and also compared to the free base molecule. Prolonged exposure of the prasugrel HCl salt to air and moisture results in some degradation which WO2006135605 solves by using air and moisture impervious gas-inerted blister packs. EP 1298132 and EP 1728794 describe the process of manufacturing tablets and capsules by using lactose as diluent, in an amount between 51 and 62%.

WO2004098713 discloses the use of over 90% of a diluent, preferably microcrystalline cellulose.

WO2008069262 describes the use of film layers containing one or more film coating base agents selected from polyvinyl alcohols, sodium carboxymethylcelluloses and pullulans for the improvement of prasugrel stability.

WO2008072532 discloses the use of water-soluble polymers such as hydroxypropyl methyl cellulose, hydroxypropyl cellulose or polyvinylpirrolidone in order to improve prasugrel stability.

WO2008072534 reports on solving the problem of content evenness by applying mannitol or lactose which, when examined under specific conditions, have a particle size distribution having a particle diameter at 90% cumulation of 80-300 micrometers.

Lactose is known to be able to interact with some pharmaceutically active ingredients, especially in the presence of water, and to hasten its degradation. Microcrystalline cellulose is chemically inert towards prasugrel, but is somewhat hygroscopic, which again increases the amount of moisture and the possibility of interactions between lactose and the active ingredient.

Owing to its very appropriate compression characteristics lactose is undoubtedly a diluent of the widest use, but it still exhibits some objectionable properties. It browns frequently in the environment displaying high relative humidity (more than 80 %). Moreover, this process is accelerated by heat and is not reproducible with respect to the particular lactose kind (content of micro-impurities may be concerned). When the active ingredients including an amine group are applied, Maillard's reaction [L. C. Maillard: Compt. Rend. 154,66 (1912)] accelerated by alkaline agents may in some cases take place and bring a medicinal product to get brown including a decrease of its active ingredient content.

It has now surprisingly been found out that the stability as well as the content eveness problem can be solved by using dry technological processes without using lactose or mannitol.

### DESCRIPTION OF THE FIGURES

Fig. 1 shows a powder X-ray diffraction pattern of amorphous prasugrel base.
Fig. 2 shows a FT-IR spectrum of amorphous prasugrel base.
Fig. 3 shows a powder X-ray diffraction pattern of prasugrel base form II.

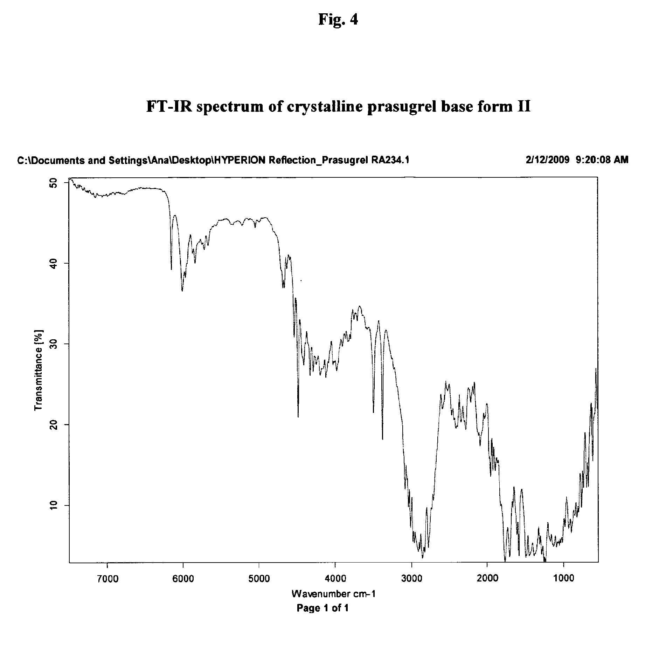

Fig. 4 shows a FT-IR spectrum of prasugrel base form II.

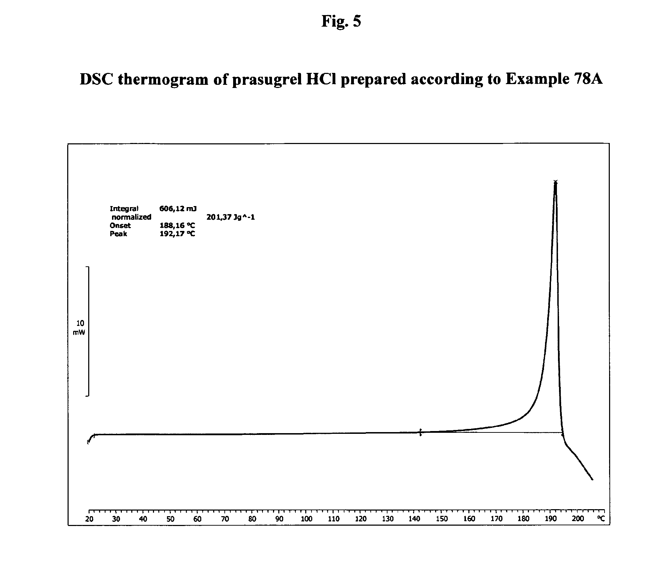

Fig. 5 shows a DSC thermogram of prasugrel HCl prepared according to Example 78A

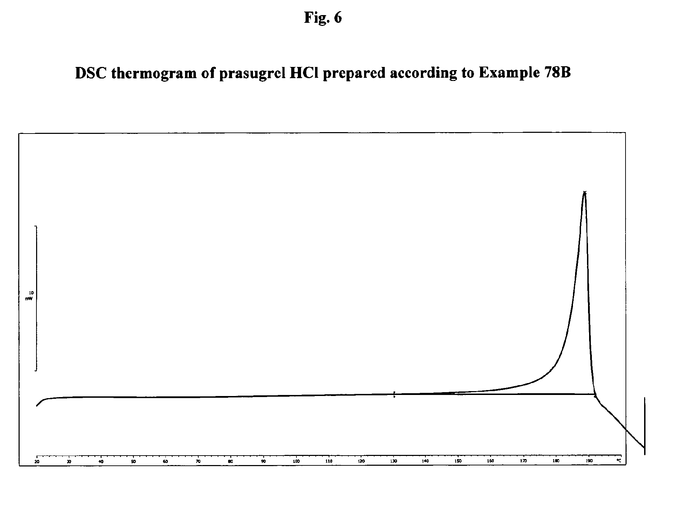

Fig. 6 shows a DSC thermogram of prasugrel HCl prepared according to Example 78B

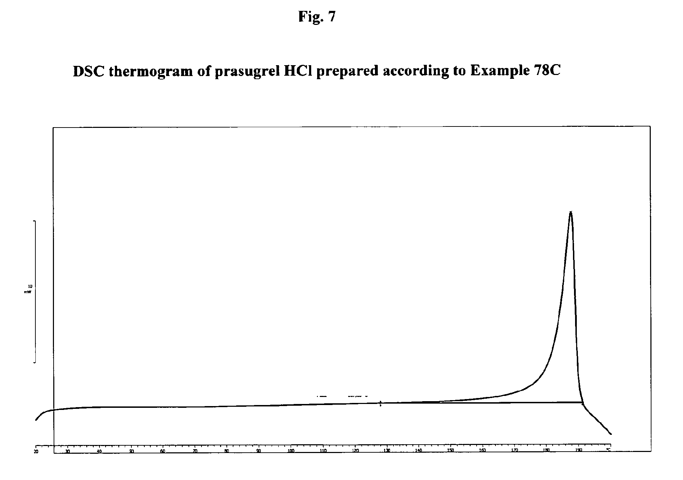

Fig. 7 shows a DSC thermogram of prasugrel HCl prepared according to Example 78C

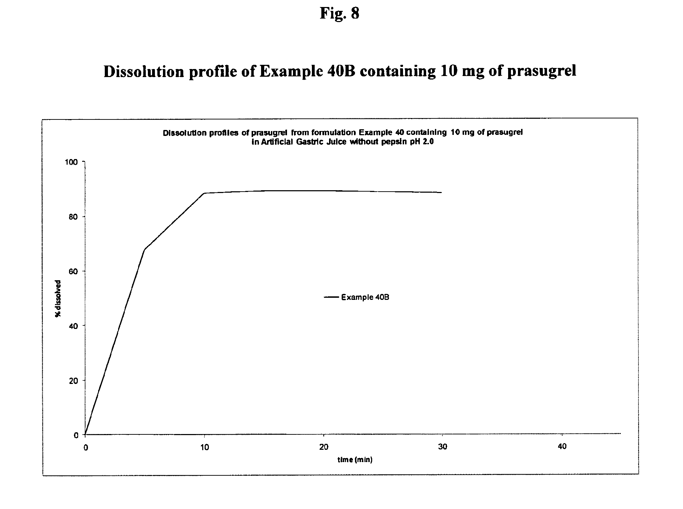

Fig. 8 shows a dissolution profile of solid composition obtained from example 40B.

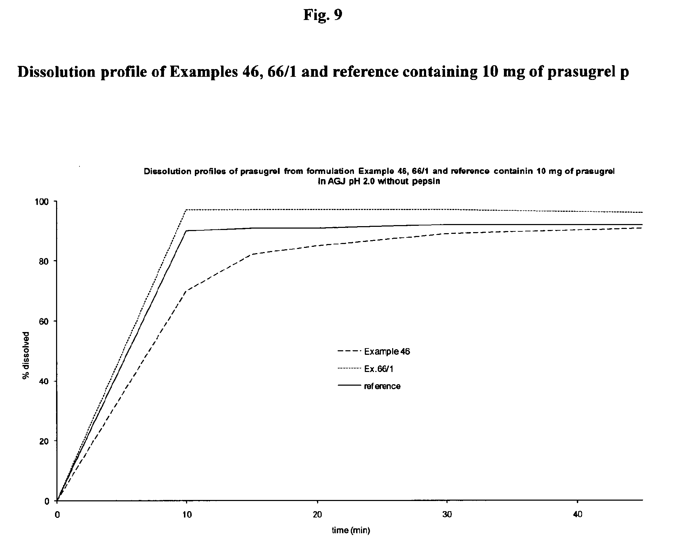

Fig. 9 shows a dissolution profile of solid composition obtained from examples 46, 66/1 and reference tablets containing 10 mg of prasugrel.

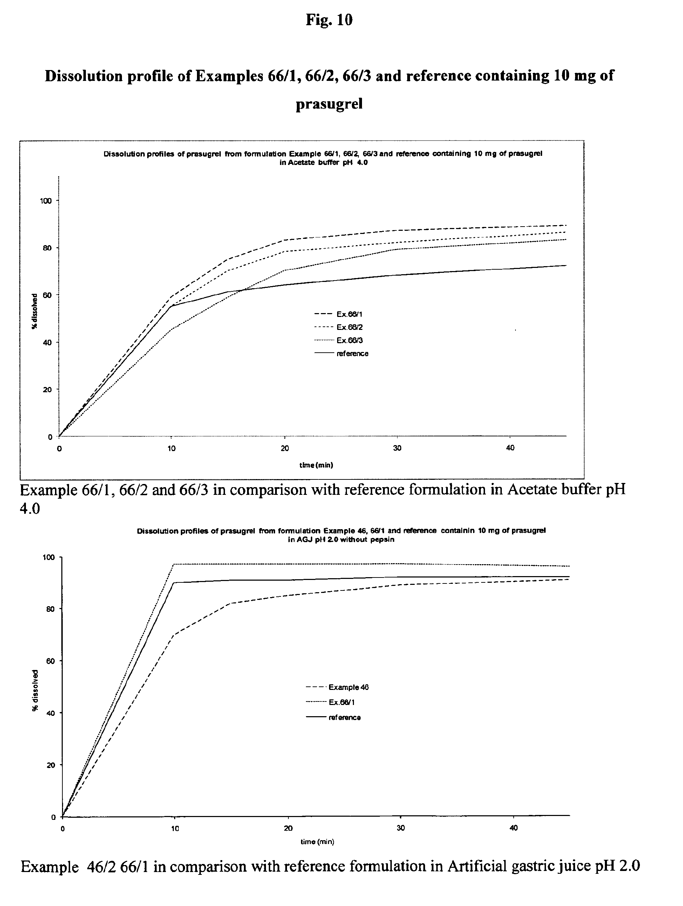

Fig. 10 shows a dissolution profile of solid composition obtained from examples 66/1, 66/2, 66/3 and reference tablets containing 10 mg of prasugrel.

### DETAILED DESCRIPTION OF THE INVENTION

The objective of the present invention is to develop a new, stable pharmaceutical composition comprising prasugrel or its pharmaceutically acceptable salts as the active pharmaceutical ingredient whose manufacturing process is simple, straightforward, and may be conducted using standard formulation methods and pharmaceutical additives. Pharmaceutical composition of prasugrel can be in the form of uncoated or coated tablets with immediate release of active agent or "oro dispersible tablets" (ODT), or micro tablets or capsules filled in with granulate or pellets or microtablets or prolonged release tablets. Immediate release means that at least 75% of prasugrel dose incorporated into said dosage form is released in less than 45 minutes when in vitro release test using USP apparatus 2 (paddle) was used (dissolution medium: 900 ml of artificial gastric juice (AGJ) without pepsin with pH =2, 37°C). ODT preparations are according to present invention designated as those having disintegration time of less than 1 minute, preferably of less than 45 seconds. Prolonged release preparations are those which release less than 50% of prasugrel from the preparation after 2 hours when in vitro release test using USP apparatus 2 (paddle) was used (dissolution medium: 900 ml of artificial gastric juice (AGJ) without pepsin with pH =2, 37°C).

Surprisingly it has now been found out that stable pharmaceutical compositions comprising prasugrel base or its pharmaceutically acceptable salts, can easily be prepared by water free, preferably solvent free pharmaceutical processes (i.e. processed in the absence of water, preferably in the absence of a solvent) and without using lactose in the pharmaceutical composition.

According to an aspect of the present invention, there is provided a process of forming a compressed mixture comprising
a.) an effective amount of prasugrel base or its pharmaceutically acceptable salt,
b.) pharmaceutically acceptable additives suitable for the preparation of solid oral dosage forms, characterized in that lactose is not used,
   by solvent free technological methods comprising the steps of
   i.) optionally grinding the active agent and pharmaceutical additives, mixing them, or mixing them first and grinding them together,
   ii.) subjecting a homogenous mixture of the active agent and additives to compression, the average particle size of prasugrel or its pharmaceutically acceptable salt being in the range of 0.2 µm to 150 µm.

According to the invention there is provided a process of forming a core of solid oral dosage form, preferably in the absence of water, more preferably in the absence of solvent, in particular in the absence of solvent selected from one or more of water, dioxane, toluene, acetonitrile, tetrahydrofurane, ethyl acetate, ethanol, methanol and mixtures thereof,
said solid oral dosage form comprising
a.) an effective amount of prasugrel base and/or its pharmaceutically acceptable salt(s) having average particle size in the range of 0.2 µm to 150 µm,
b.) pharmaceutically acceptable additives suitable for the preparation of solid oral dosage forms, wherein lactose is not used,
   by solvent free technological methods, comprising the steps of
   i.) optionally grinding the active agent (the active agent comprising or consisting essentially of or consisting of prasugrel base and/or its pharmaceutically acceptable salt(s), preferably the active agent consisting essentially of or consisting of prasugrel base and/or its pharmaceutically acceptable salt(s)) and pharmaceutical additives, mixing them, preferably mixing them after grinding,
      or mixing them (active agent and pharmaceutical additives) first and grinding them together, preferably grinding them during and/or after mixing,
      preferably carrying out step i) such that a homogenous mixture results,
   ii.) subjecting a mixture of optionally ground active agent and additives, preferably a homogenous mixture of the optionally ground active agent and additives to compression,
      preferably carrying out step i) or steps i) and ii) without adding water in unbound form, in particular without adding water, more preferably carrying out step i) or steps i) and ii) without adding unbound solvent, in particular without adding solvent, especially without adding solvent or solvent in unbound form selected from or comprising one or more of water, dioxane, toluene, acetonitrile, tetrahydrofurane, ethyl acetate, ethanol, methanol and mixtures thereof.

According to a preferred embodiment, lactose and mannitol are not added during the process of forming said solid oral dosage form.

According to a further preferred embodiment, said solid oral dosage form is free of or substantially free of lactose and free of or substantially free of mannitol.

According to a preferred embodiment of the invention, the solid oral dosage form may comprise or consist of a compressed mixture, said compressed mixture comprising or consisting of an effective amount of prasugrel base and/or its pharmaceutically acceptable salt(s) and pharmaceutically acceptable additive(s) suitable for the preparation of solid oral dosage forms, wherein preferably lactose and mannitol are not or substantially not present, and optionally no coating or at least one coating. Preferably lactose and mannitol are not or substantially not present in the compressed mixture.

According to one embodiment of the invention, the compressed mixture can be in the form of a core or a tablet core in the solid oral dosage form or the pharmaceutical composition of the invention, or can form part of said core or tablet core. According to one preferred embodiment, at least one coating can be applied on the (tablet) core. In the following paragraph, reference to "the compressed mixture" is therefore also to be understood as reference to "the (tablet) core", according to a preferred embodiment.

The compressed mixture is prepared by solvent-free technological methods and processes, preferably as described herein. It is also preferred that the compressed mixture is prepared without adding water in unbound form, in particular without adding water, preferably without adding unbound solvent, in particular without adding solvent, especially without adding solvent or solvent in unbound form selected from or comprising one or more of water, dioxane, toluene, acetonitrile, tetrahydrofurane, ethyl acetate, ethanol, methanol and mixtures thereof. It has been surprisingly found that this provides advantageous solid oral dosage forms / pharmaceutical compositions according to the invention.

According to one embodiment of the invention, the at least one coating used in the solid oral dosage form (the pharmaceutical composition, respectively) and/or the coating fluids used for applying said at least one coating can comprise solvent, in particular water, or can be free of or essentially free of solvent, in particular water. Preferably, the at least one coating is free of or essentially free of lactose and/or mannitol.

The compression force under ii.) is in the range of 1-50 kN, and depends on whether step ii.) is the final step of the process (i.e. direct tableting) or if further steps are needed to prepare the final oral solid dosage form.

In one of the embodiments compressing under (ii) can be performed by state of the art methods of dry granulation methods like roller compaction and/or slugging. In this case the compression force under ii.) is smaller, from 1-40 kN, preferably 3-30 kN, to yield softer comprimates. The obtained compacted material is ground and optionally sieved to get a granulate with appropriate physical properties such as particle size, particle size distribution, flowability and compressibility. In the next step the obtained granulate is mixed with at least one additional pharmaceutical additive selected from disintegrants, diluents, glidants, lubricants and optionally other additives such as binders, solubility enhancers, stabilisers, antioxidant agents, surfactants, adsorbents, and compressed into tablets.

In another embodiment, compressing under (ii) can be performed by direct compression of a homogenous mixture of active agent and additives. In this case, the comprimate obtained under ii.) is the final oral solid dosage form, and the compaction force under ii.) need be higher, preferably from 5 to 50 kN, depending on the size and shape of the tablet.

Optionally the solubility enhancer used is ground together with prasugrel base or its pharmaceutically acceptable salt. Preferably, the acidic solubility enhancer is ground together with prasugrel base, to a particle size of between 10 nanometers and 150 micrometers, preferably between 0.2 micrometers and 30 micrometers, most preferably between 1 and 10 micrometers. High energy grinding such as ball milling or air jet milling is preferred.

In a special embodiment of the present invention a ground active ingredient and solid additives are subjected to solvent free granulation using at least one additive having melting or glass transition point or softening point below 180°C, preferably below 150°C and even more preferably below 100°C by the processes and equipment known from the state of the art such as melt extrusion, melt granulation or melt coating. Glass transition point of the melted additive can be decreased if needed by the addition of suitable plasticizers. The obtained granulate can be sieved if needed and mixed with suitable additives selected from binders, disintegrants, diluents, lubricants, glidants and optionally other additives such as binders, solubility enhancers, stabilisers, antioxidant agents, surfactants, adsorbents and compressed into tablets.

Marumarizers or single or twin screw extrudors can be used for hot melt extrusion, where the melting or softening of at least one additive is achieved by increased pressure and/or external heating of the wall of extruder.

Melt granulation can be performed in shear mixers such as low or high shear mixers/granulators optionally equipped with heating/cooling jackets or in fluid bed granulators such as those produced by Glatt, Niro Aeromatic or Hüttlin. Granulation can be achieved by spraying of melted binder optionally admixed with other additives having stabilizing and/or solubilising effect on to active agent or on the homogenous mixture of the active agent, diluent and at least one further pharmaceutical additive selected from disintegrant, antioxidant, and solubilising agent.

In a special embodiment the active agent can be dispersed, preferably dissolved in the melted additive and the dispersion is sprayed onto the mixture of diluent and optionally other additives. In an another embodiment active agent dispersed in the melted pharmaceutical additive with optional other pharmaceutical additives being homogenously dispersed into the above dispersion is spray coated by the processes known from the state of the art such as fluid bed coating onto the cores such as neutral pellets, neutral granulate or neutral crystals with the average particle size in the range 0.1 to 1.5 mm, preferably 0.2 to 1.2 mm.

Term dispersed according to present invention means that the substance such as active agent or pharmaceutical additive or the mixture thereof is suspended, dissolved and/or dissolved in the melted or softened pharmaceutical additive having low melting or softening point.

In a further embodiment melt granulation in the shear mixer can be achieved by *in situ* melting of at least one additive by external heating of the jacket of the mixer wall or by the heat produced by the friction among particles during their mixing.

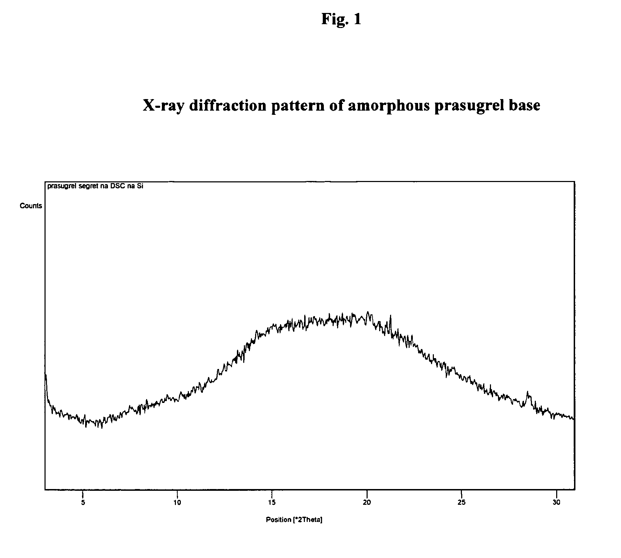

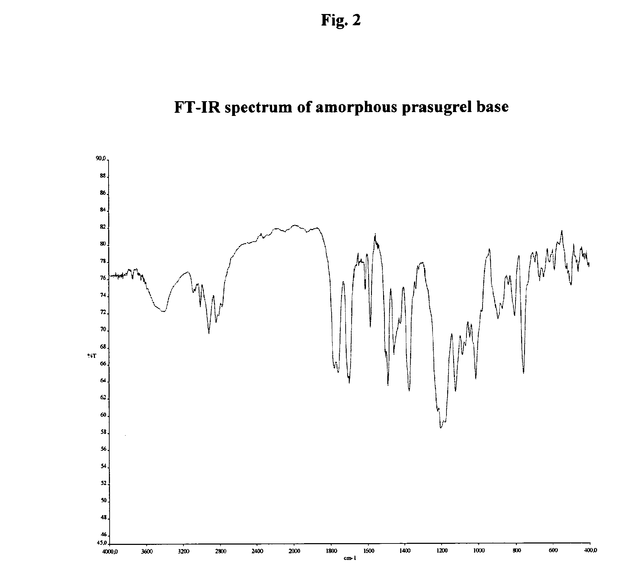

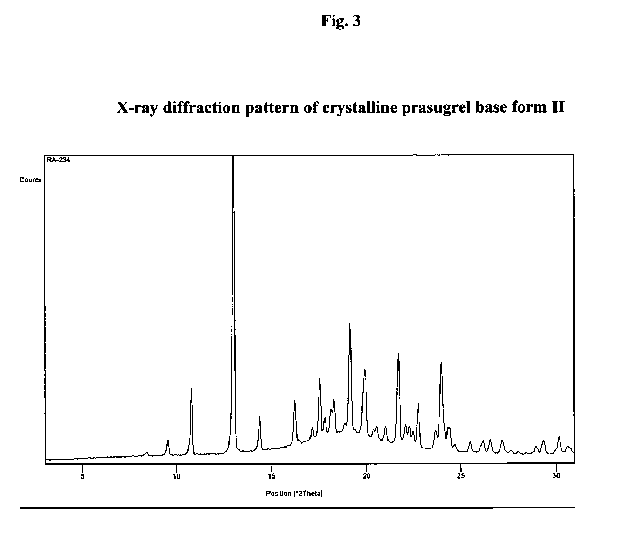

Prasugrel base (1) as used in the preparation of prasugrel salts or pharmaceutical compositions of the present invention may be produced according any of the reported manufacturing method, including the one described by this patent application, or any method obviously employed by the average person skilled in the art. In the present invention, amorphous prasugrel base is prepared by precipitation from organic solvents such as alcohols, ethers, nitriles, aromates, ketones, ethers etc. Examples of such solvents are dioxane, toluene, esters, acetonitrile, tetrahydrofurane, ethyl acetate, ethanol and mixtures thereof. For its X-ray diffraction pattern see Figure 1. Also any other known crystalline form of prasugrel salts or base may be applied, including the new polymorphic form II of prasugrel base described by the present invention. For its X-ray diffraction pattern see Figure 2 and for its FT-IR spectrum Fig. 3. (BRUKER Optics, FT-IR microscope Hyperion 300).

Amorphous form of prasugrel base or prasugel salts can according to one aspect of the present invention be produced by grinding of the salt sample in a ball mill alone or optionally in admixture with at least one pharmaceutically acceptable additive selected from diluents, surfactants, disintegrants, stabilizers, crystallization inhibitors and/or binders, or a mixture thereof. The mechanical force exerted on the particle surface during grinding leads to particle size reduction. Grinding can be performed by any milling process known in the art, for example using a ball mill (planetary ball mill or mixer mill), hammer mill, bead mill, disc mill, ultrasonic mill, torus mill, impact mill, vibration mill, pin mill or air jet mill. Amorphous forms of pure prasugrel base or prasugrel salts can in an another aspect be produced by other techniques known from the state of the art such as but not limited to lyophilization, spray drying, fast cooling of melted active agent or fast cooling of hot saturated solutions of active agent. In still another aspect of present invention amorphous form of prasugrel or its salts can be obtained by producing solid dispersion, solid solution, complex, adsorbates and precipitates of active agent with and/or on and/or in the carrier. Water soluble, water insoluble hydrophilic pharmaceutical additve can be used as carriers. In case of complexes carriers such as cyclodextrins having 6-9 monomeric units in the ring such as (α, β, γ or δ cyclodextrin), or polymers having free carboxylic and/or sulphonic groups in the molecules and having pH of aqueous solution or suspension in concentration in the range of 2 to 10 weight/volume % below 5.5, preferably below 5 such as polymethacrylates, carrageenans, xantanes can be used.

In case prasugrel base of increased purity is desired, the preparation of prasugrel salts may also be used as a purification step, and the prasugrel salt then converted to prasugrel base.

X-ray powder diffraction patterns were obtained by Phillips PW3040/60 X'Pert PRO diffractometer; CuK_{α} radiation 1,541874 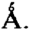

Prasugrel base form II obtained by the present invention is characterized by the following 2-theta degrees: ±0.2 (Table 1), and the following peak in the FT-IR spectrum: 3384.0, 2841.1, 2395.4, and 2081.1.

**Table 1**

| No. | Pos. [°2Th.] | d-spacing [A] | Rel. Int. [%] |
|---|---|---|---|
| 1 | 10.8 | 22.6 | 8.20 |
| 2 | 13.0 | 100.0 | 6.81 |
| 3 | 16.2 | 18.7 | 5.47 |
| 4 | 17.5 | 26.3 | 5.07 |
| 5 | 19.1 | 44.1 | 4.64 |
| 6 | 19.9 | 28.6 | 4.46 |
| 7 | 21.7 | 34.9 | 4.10 |
| 8 | 23.9 | 31.0 | 3.72 |

The prasugrel hydrochloride prepared according to the present invention has a melting point between 188.9 °C and 191.2°C, as measured by the capillary method where the temperature gradient applied was 10°C/ min.

The pharmaceutically acceptable salts of 2-acetoxy-5-(alpha-cyclopropylcarbonyl-2-fluorobenzyl)-4,5,6,7-tetrahydrothieno[3,2-c]pyridine may be, for example, hydrohalogenic acid salts such as hydrofluoride, hydrochloride, hydrobromide or hydroiodide; nitrate; perchlorate; sulfate; phosphate; C1-C4 alkanesulfonates optionally substituted by halogens such as methanesulfonate, trifluoromethanesulfonate, ethanesulfonate; C6-C10 arylsulfonates optionally substituted by C1-C4 alkyl groups such as benzenesulfonate or p-toluenesulfonate; C1-C6 aliphatic acid salts such as acetate, malate, fumarate, succinate, citrate, tartarate, oxalate or maleate; amino acid salts such as glycine salt, lysine salt, arginine salt, omitine salt, glutamic acid salt or aspartic acid salt. The preferred compound is prasugrel base.

The solubility of prasugrel base and some of its pharmaceutically acceptable salts in different dissolution media (in mg/mL) at room temperature and 37°C is given in Table 2.
The equilibrium solubility of prasugrel base and some of its salts were checked in aqueous solutions at room temperature and 37 °C with shaking flask method for 24 hours (Heidoplh Incubator 1000, Unimax 1010). In comparison with prasugrel base, all salts are better soluble in lower pH values. In higher pH values the solubility decreases and at pH 6.8 the compound is slightly soluble to practically insoluble.
It is assumed that the solubility of prasugrel base in aqueous solutions having different pH depends on its dissociation constant pKa of 5.40 and the pH of the medium.

The equilibrium solubility of hydroiodide and hydrobromide salt is comparable with solubility of hydrochloride salt of prasugrel.

**Table 2**

| Compound | Medium | Solubility (mg/mL) room temp | Solubility (mg/mL) at 37°C |
|---|---|---|---|
| Prasugrel base | AGJ (without pepsin) pH 1.2 | 1.54 | 3.23 |
| | Acetate Buffer solution pH 4.0 | 0.03 | 0.03 |
| | Phosphate Buffer solution pH 6.8 | 0 | 0 |
| | AGJ (without pepsin) pH 1.2 | 5.00 | 4.43 |
| Prasugrel hydrochloride | Acetate Buffer solution pH 4.0 | 0.07 | 0.08 |
| | Phosphate Buffer solution pH 6.8 | 0 | 0 |
| | AGJ (without pepsin) pH 1.2 | 2.600 | 3.660 |
| Prasugrel hydroiodide | Acetate Buffer solution pH 4.0 | 0.132 | 0.166 |
| | Phosphate Buffer solution pH 6.8 | 0.00316 | 0.0018 |
| | AGJ (without pepsin) pH 1.2 | 3.924 | 4.098 |
| Prasugrel hydrobromide | Acetate Buffer solution pH 4.0 | 0.138 | 0.153 |
| | Phosphate Buffer solution pH 6.8 | 0.003 | 0.009 |

When 2-acetoxy-5-(alpha-cyclopropylcarbonyl-2-fluorobenzyl)-4,5,6,7-tetrahydrothieno[3,2-c]pyridine or a pharmaceutically acceptable salt thereof, is allowed to stand so that it is open to the atmosphere, it may become hydrated by absorption of water or adsorption of water. Such hydrated compounds are included in the present invention. 2-Acetoxy-5-(alpha-cyclopropylcarbonyl-2-fluorobenzyl)-4,5,6,7-tetrahydrothieno[3,2-c]pyridine or a pharmaceutically acceptable salt thereof, may also absorb some kinds of organic solvents and may form solvates in some cases, and these solvates are also included in the present invention.

Furthermore, since 2-acetoxy-5-(alpha-cyclopropylcarbonyl-2-fluorobenzyl)-4,5,6,7-tetrahydrothieno[3,2-c]pyridine has an asymmetric carbon atom, optical isomers exist based on the asymmetric carbon atom. These optical isomers are also included in the present invention.

According to a further aspect, the present invention provides a pharmaceutical composition obtainable according to any of the processes as set forth in the present application.

According to another aspect, the present invention provides a pharmaceutical composition preferably prepared in the absence of water, more preferably prepared in the absence of a solvent, especially prepared in the absence of a solvent selected from or comprising one or more of water, dioxane, toluene, acetonitrile, tetrahydrofurane, ethyl acetate, ethanol, methanol and mixtures thereof, by compressing a mixture, preferably a homogenous mixture, said mixture comprising a prasugrel base and/or a pharmaceutically acceptable salt thereof and pharmaceutically acceptable additives suitable for the preparation of solid oral dosage forms, wherein preferably lactose and mannitol are not used, in particular wherein lactose and mannitol are not added to said mixture to be compressed.

According to an embodiment, the mixture to be compressed is free of or essentially free of lactose and mannitol and can be free of added water, preferably free of added solvent, more preferably free of added solvent selected from or comprising one or more of water, dioxane, toluene, acetonitrile, tetrahydrofurane, ethyl acetate, ethanol, methanol and mixtures thereof.

According to a further embodiment, the pharmaceutical composition of the tablet core can be prepared in the absence of unbound water, more preferably prepared in the absence of unbound solvent, especially prepared in the absence of unbound solvent selected from or comprising one or more of water, dioxane, toluene, acetonitrile, tetrahydrofurane, ethyl acetate, ethanol, methanol and mixtures thereof,

Moreover, the present invention provides a pharmaceutical composition comprising or consisting of a compressed mixture, preferably a compressed homogenous mixture, said compressed mixture comprising a prasugrel base and/or a pharmaceutically acceptable salt thereof and pharmaceutically acceptable additives suitable for the preparation of solid oral dosage forms.

A pharmaceutical composition can be preferably free of added water, in particular free of added solvent, more preferably free of an added solvent selected from or comprising one or more of water, dioxane, toluene, acetonitrile, tetrahydrofurane, ethyl acetate, ethanol, methanol and mixtures thereof, and/or a pharmaceutical composition is free of added lactose and optionally free of added mannitol. In particular, a pharmaceutical composition is free or substantially free of lactose and can be free or substantially free of mannitol.

The term "free of added water" ("free of added solvent") means in particular that during the preparation process, e.g. of a tablet core of pharmaceutical composition or mixture to be compressed, no water (solvent) has been added as such.

Preferably at least one or each of the ingredients used for preparing a pharmaceutical composition of the present invention can have a content of unbound water, preferably a total water content, of below 4 weight%, preferably below 3 weight%, based on the total weight of the respective one of said ingredients, respectively. Preferably at least one or each of the ingredients used for the preparation process of the pharmaceutical composition can be dried such that they are free or substantially free of water, in particular free or substantially free of solvent.

The term "free of added lactose" means that during the preparation process no lactose has been added as such. The term "free of added mannitol" means that during the preparation process no mannitol has been added as such. Lactose and mannitol are preferably not or substantially not present in a pharmaceutical composition of the present invention. The "term lactose and mannitol being substantially not present in a pharmaceutical composition" can mean that lactose is preferably present in a pharmaceutical composition of the present invention in an amount of less than 0.05 weight%, and mannitol is preferably present in said pharmaceutical composition of the present invention in an amount of less than 0.05 weight%, each based on the total weight of the pharmaceutical composition.

According to another aspect, pharmaceutical compositions of the present invention for use in the treatment, alleviation and/or prevention of thrombosis and/or for use as an adjunct to percutaneous coronary intervention procedures are provided.

According to yet another aspect, the present invention provides the use of a pharmaceutical composition of the present invention, for the preparation of a medicament for the treatment, alleviation and/or prevention of thrombosis and/or for the alleviation of percutaneous coronary intervention procedures.

Further, the solid pharmaceutical preparation of this invention may comprise various ingredients, according to necessity, such as pharmacologically acceptable lubricants, glidants, binders, emulsifiers, buffering agents, crystallization inhibitors, disintegrants, antioxidant agents, solubility enhancers, taste and odor improving agents and/or diluents.

The "lubricant" used can be, for instance, stearic acid; a metal salt of stearic acid such as calcium stearate or magnesium stearate, talc; colloid silica; a wax variety such as beads wax or spermaceti; boric acid; adipic acid, a sulphate such as sodium sulphate; glycol, fumaric acid, stearyl sodium fumarate; sucrose aliphatic acid ester, sodium benzoate, D,L-leucine; a lauryl sulphate such as sodium lauryl sulphate or magnesium lauryl sulphate; a kind of silicic acid such as silicic anhydride or silicic acid hydrate; or a starch derivative such as corn starch, potato starch, α-starch or dextrin, etc. Preferred item is a metal salt of stearic acid.

Glidants can be selected from talc, colloidal silica such as Aerosil 200 or Aerosil R972 or silicates.

Diluents can be selected from dextran, starch and its derivatives such as corn starch, pregelatinized starch, particularly low moisture pregelatinized starch such as Starch 1500 LM obtainable from Colorcon, coprocessed starch and pregelatinized starch such as StarCap 1500 obtainable from Colorcon, powdered cellulose, e.g. commercially available as Arbocel^{®} M80, Arbocel^{®} P290 or Arbocel^{®} A300 from JRS Pharma, microcrystalline cellulose, particularly low moisture microcrystalline cellulose such as Avicel^{®} PH103, Avicel^{®} PH112, Avicel^{®} PH113 or Avicel^{®} PH200 LM from FMC BioPolymer and such as Vivapur^{®} 103, Vivapur^{®} 112 , Vivapur^{®} 14, Vivapur^{®} XLM 200, Emcocel^{®} 50M or Emcocel^{®} XLM 200 from JRS Pharma, silicified microcrystalline cellulose, particularly low moisture silicified microcrystalline cellulose such as Prosolv^{®} SMCC 90 LM from JRS Pharma, granulated and or spray dryed xylitol such as Xylitab^{®} 300 obtainable from Danisco or coprocessed xylitol such as Xylitab^{®} 100, Xylitab^{®} 200 obtainable from Danisco and directly compressible xylitol such as Xylisorb^{®} 300, Xylisorb^{®} P 300 DC obtainable from Roquette, compressible sugar, sucrose, fructose or coprocessed fructose and starch such as Advantose^{®} FS 95 obtainable from SPI Pharma, isomalt, metal salts of phosphoric acid such ad calcium hydrogenphosphate in anhydrous or hydrated state. Low moisture diluents being preferred. In a special embodiment of the present invention coprocessed diluents on the basis of carbohydrates such as xylitol, lactitol, isomalt, maltodexrin, maltitol can be prepared by granulating of diluents by binders selected from another carbohydrate derivative such as soluble sorbitol, maltitol, lactitol, polydextrose, sodium carboxymethylcelullose or other polymeric water soluble binders such as cellulose ethers, povidone, copovidone, polyethyleneglycole or the like.

In order to achieve good processibility during compression, the particle size of the diluents is important. The average particle size of diluents used for direct compression or in the extragranular phase can be in the range from 50 to 500 µm, preferably 75 to 400 µm. Diluents especially designed for direct compression having physically modified particles obtained by state of the art processes such as spray drying and/or granulation are especially preferred for use in the extragranular phase. Coprocessed diluents having improved compressibility and flowability such as silicified microcrystalline cellulose alone or coprocessed with additional pharmaceutical additives such as disintegrants, glidants and lubricants e.g. different Prosolv types obtainable from Penwest, directly compressible xylitol such as Xylitab^{®} 100, Xylitab^{®} 200 obtainable from Danisco, coprocessed fructose and starch such as Advantose^{®} FS 95 obtainable from SPI Pharma can be used. In case of diluents used intragranularly, the average particle size can be in the range from 10 to 200 µm, preferably 20 to 150 µm. The average particle size is determined by laser diffraction method such as by Malvern instrument.

The "binder" used can be, for instance, hydroxypropyl cellulose, hydroxypropyl methyl cellulose, polyvinyl pyrrolidone, polyethylene glycol, or an excipient (e.g. white sugar, glucose, sorbitol, etc. sugar derivative such as e.g. polydextrose, maltodextrin; a starch derivative such as corn starch, potato starch, α-starch or dextrin; a cellulose derivative such as crystalline cellulose; gum arabic; dextran; or pullulan etc. organic-type pharmaceutical additives, or light silicic anhydride, synthetic aluminium silicate, calcium silicate or magnesium metasilicate-aluminate, etc. silicate derivatives; calcium hydrophosphate etc. phosphates; calcium carbonate etc. carbonates; calcium sulphate etc. sulphates or other inorganic type pharmaceutical additives) and similar compounds. Preferable item among them is hydroxypropyl cellulose or hydroxypropyl methylcellulose.

Solubility enhancers are inorganic or organic acids such as (-)-(2S,3S)-tartaric acid, (+)-(2S,3S)-di-O-benzoyltartaric acid, (+)-(2S,3S)-di-O-(4-methylbenzoyl)tartaric acid, (-)-*L-*phenylalanine, benzenesulfonic acid, cyclohexanesulfamic acid, naphthalene-2-sulfonic acid, sebacic acid, (+)-camphor-10-sulfonic acid, p-toluenesulfonic acid, ethanesulfonic acid, methanesulfonic acid, adipic acid, pimelic acid, maleic acid, fumaric acid, citric acid, malic acid, succinic acid, glutaric acid, malonic acid, galic acid, ferrulic acid etc. Preferably the acidic solubility enhancer is present in the pharmaceutical composition in an amount between 0.05 weight percent and 25 % w/w, preferably in an amount between 0.25 and 5 % w/w. Optionally the solubility enhancer is ground together with prasugrel base or its pharmaceutically acceptable salt. Preferably, the acidic solubility enhancer is ground together with prasugrel base, to a particle size of between 10 nanometers and 150 micrometers, preferably between 0.2 micrometers and 30 micrometers, most preferably between 1 and 10 micrometers.

Different types of "disintegrants" such as NVP water-swellable polymers, crospovidone, croscarmellose sodium, carmellose sodium or calcium, cellulose derivatives such as low substituted hydroxypropyl cellulose, sodium starch glycolate, alkali and earth alkali salts of carboxymethyl cellulose and ionic resins such as potassium salt of polacrilin or other pharmaceutically acceptable disintegrants or their combinations can be used in the invention. An "NVP water- swellable polymer" is an insoluble, swellable homo- or heteropolymer containing N- vinylpyrrolidone, e.g. N-vinyl-2-pyrrolidone. Preferable disintegrant is croscarmellose sodium.

The disintegrants can be added to other pharmaceutical additives according to the process used in the state of the art, either in the process of granulating (intragranularly) and/or in the preparation of the compression mixture (extragranularly) or in both phases.

The binder for melt granulation having melting point below 130°C, preferably below 100°C and most preferably below 80°C can be selected from the group of polyethleneglycols with molecular weights of 1.500 to 20.000 such as PEG 4.000, PEG 6.000, poloxamers with molecular weights in the range 5.000 to 20.000 such as Poloxamer 188, Poloxamer 237, Poloxamer 338 or Poloxamer 407 commercially available as Lutrol^{®} or Pluronic^{®} types, C₈-C₁₈ fatty acid esters of polyoxylglicerides of Gelucire^{®} type, C₈-C₁₈ fatty acid esters of polyethylene glycol, sugar esters such as sucrose stearate, sucrose palmitate or sucrose laurate. The mass ratio beetween the active substance (prasugrel) and binder for melt granulation in the granulate is in the range 1:10 to 10:1, preferably 2.5:10 to 10:2.5, most preferably 5:10 to 10:5.

The adsorbent can be selected from anorganic materials having a specific area of more than 25 m²/g preferably of more than 50m²/g, such as colloidal anhydrous silica sold under trade name Aerosil 200, bentonite, zeolite, porous silicone dioxide sold under trade name Sylysia 350 or magnesium aluminometasilicate sold under trade name Neusilin, and types such as UFL2 and US2.

The "stabiliser" used can be, for instance, a para-oxybenzoic acid ester such as methyl paraben or propyl paraben; an alcohol derivative such as chlorobutanol, benzyl alcohol or phenyl ethyl alcohol; benzalkonium chloride; a phenol variety such as phenol or cresol; thimerosal; dehydro acetic acid; or sorbic acid, etc.

Antioxidant agents can be butylated hydroxyanisole (BHA), 2,6-di-tert-butyl-4-methylphenol (BHT), propyl gallate, coffee acid, rutin quercetin, ascorbic acid or its metal salts, citric acid, edetate disodium and calcium metabisulphite, with BHA, propyl gallate and combinations thereof.

Solubility enhancers can be selected from anionic, cationic, ampholitic or nonionic surfactants. The cationic surfactants are selected from the group consisting of organic quaternary ammonium halides, R₄N⁺Cl⁻, cetrimide, a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula [C₆H₅CH₂N⁺(CH₃)₂R]Cl⁻, where R represents an alkyl selected from C₈H₁₇ to C₁₈H₃₇. The anionic surfactant is selected from the group consisting of organic sulphonates (RSO₃⁻) or sulphates (ROSO₃⁻), potassium laurate, CH₃(CH₂)₁₀COO-K⁺, and sodium lauryl sulphate, CH₃(CH₂)₁₁SO₄-Na⁺. The most preferred anionic surfactant is sodium lauryl sulphate.

The ampholytic surfactants are selected from sulfobetaines, RN⁺(CH₃)₂(CH₂CH₂SO₃-), N-Dodecyl-N,N-dimethylbetaine, C₁₂H₂₅N⁺(CH₃)₂CH₂COO⁻.

The nonionic surfactants are selected form the surfactants containing hydroxyl or polyoxyethylene (OCH₂CH₂O⁻) groups, and more preferably it is selected from polyoxyethylated glycol monoethers, cetomacrogol, sorbitan esters (Spans) and polysorbates (Tweens), or from polyoxyethylene-polyoxypropylene copolymers (poloxameres), or from the group of esters such as dioctyl sulphosuccinate and/or sucrose esters with fatty acids such as sucrose stearate or the like.

The "taste improving and odor improving agent" can be, for instance, a sweetener such as saccharin sodium or aspartame; an acidifier agent such as citric acid, malic acid or tartaric acid; or an aromatic substance such as menthol, lemon or orange.

The amount blended of the compound of the above formula (1) or a pharmacologically acceptable salt thereof in the total amount of the solid medicinal preparation is not particularly restricted, for instance, an amount of 1.0 to 30.0 % in weight is suitable for the total amount of solid medicinal preparation (preferably 2 to 20.0 % by weight).

The pharmaceutical compositions may further comprise 0.5 to 5.0 % by weight of a lubricant (preferably 0.5 to 3.0 % by weight) and an amount of 0.0 to 15.0 % by weight of a binder (preferably 2.5 to 10.0 % by weight), each based on the total weight of the pharmaceutical composition, which amounts are suitable for the total amount of solid medicinal preparation. The total sum of the combination of components of the formulations of the present invention is 100%.

In this invention, the solvent free method can be a direct compression method or a solvent free granulation method such as roller compaction, slugging, melt extrusion or melt granulation, the direct tableting method being preferable.

The "direct compression method" is a method of formulation wherein the raw material powder is subjected to direct compression moulding without any preagglomeration processes.

The "dry granulation method" is a method of formulation wherein the raw material powder is subjected to compression moulding into comprimate or sheet, wherein granules are produced by crushing and division of comprimates and/or sheets by a suitable method. Such methods are described in The Theory and Practice of Industrial Pharmacy (Third Edition)(Leon Lachman et al., LEA & FEBIGER 1986) or Pharmaceutical Dosage Forms: Tablets Volume 1 (Second Edition)(Herbert A. Lieberman et al.: MARCEL DEKKER Inc. 1989).

In this respect, granulation is a process to produce agglomerated primary particles with substantially uniform shape and size from powders, pellets, liquids or melts, etc. raw materials, including granulation producing final products such as granules, dispersions or powder medicines and granulation producing intermediary products for manufacturing tablets or capsules.

The granulate obtained in this manner can be classified to the required particle size and transformed into the formulation of a dispersion, powder or granules. These formulations can also be filled into a capsule to prepare encapsulated product or, after further addition of pharmaceutical additives selected from disintegrating agents and/or lubricants and/or other additives according to necessity, formulated into tablets by means of compression moulding in a tableting machine or the like. The processes of mixing or granulating, etc. are generally well known in the field of the pharmaceutical technology and the skilled in the art can select the appropriate solution. At least one layer of film coating can also be applied on the tablet.

Coating is performed using, for instance, a film coating apparatus, and the film coating base can be, for instance, a sugar coating base, a water-soluble film coating base, an intestinally soluble film coating base or a slow-release film coating base. Specially useful are the coatings having decreased permeability for gases such as oxygen and/or water vapour (moisture) based on polymers such as cellulose ethers such as hyprolose or hypromelose, block copolymer of polyvinyl alcohol and polyethylene glycol commercially obtainable under trade name of Kollicoat IR or Kollicoat protect, polyvinyl alcohol, aminoalkyl methacrylate copolymers such as Eudragit EPO, methacrylic acid copolymers such as Eudragit L, salts of carboxymethylcellulose. Further additives selected from plasticizers, pigments, colorants, and antitacking agents. The thickness of functional film coating can be in the range of 10 to 100 µm, preferably 15 to 50 µm.

The sugar coating base is white sugar, which may be combined with one or more selected from among talc, precipitated calcium carbonate, calcium phosphate, calcium sulphate, gelatine, gum arabic, polyvinyl pyrrolidone and pullulan.

A water-soluble film coating base can be, for instance, hydroxypropyl cellulose, hydroxypropyl methyl cellulose, hydroxyethyl cellulose, methylhydroxyethyl cellulose, or carboxymethyl cellulose sodium salt, etc. cellulose derivative; polyvinyl acetal diethylaminoacetate, aminoalkyl methacrylate copolymer or polyvinyl pyrrolidone etc. synthetic polymer; or pullulan etc. polysaccharide.

The intestinally soluble film coating base can be, for instance, hydroxypropyl methyl cellulose phtalate, hydroxypropyl methyl cellulose acetate-succinate, carboxymethyl ethyl cellulose, or cellulose acetate-phtalate, etc. cellulose derivative; (meth)acrylic acid copolymer L, (meth)acrylic acid copolymer LD or (meth)acrylic acid copolymer S etc., acrylic acid derivative; or shellac etc. natural substance.

The slow release film coating base can be, for instance, ethyl cellulose etc. cellulose derivative; or, aminoalkyl methacrylate copolymer RS or ethyl acrylate/methyl methacrylate copolymer emulsion etc., acrylic acid derivative, etc.

The above-mentioned coating bases can be used also in combination comprising two or more items. Further, in case of need, suitably selected ingredients can be admixed such as pharmacologically acceptable plasticisers, pharmaceutical additives, lubricants, suppressing agents, colorants and/or preservatives.

There is no particular restriction as for a plasticiser used in this invention and the skilled in the art can optionally select them. Examples of plasticisers include propylene glycol, polyethylene glycol, polypropylene glycol, glycerol and sorbitol, glycerol triacetate, diethyl phtalate and citric acid triethyl ester, lauric acid, sucrose, dextrose, sorbitol, triacetin, acetyl triethyl citrate, triethyl citrate, tributyl citrate and acetyl tributyl citrate.

The suppressing agent used in this invention can be, for instance, titanium oxide, etc.

The coloring agent used in this invention can be, for instance, water insoluble pigments such as titanium oxide, iron oxide, iron sesquioxide, yellow iron sesquioxide or yellow No. 5 aluminium lake talc, etc. or water soluble colourants.

The preservative used in this invention can be, for instance, paraben or the like.

The choice of the process and of the pharmaceutical additives is preferable to achieve the desired water content of the composition which result in highly stable final product. It has been found out that it is preferable to control unbound water (sometimes called mobile or free water) of the composition below 4 weight%, preferably below 3.5 weight% and more preferably below 3 weight% of the total weight of the composition in order to avoid undesired chemical degradation of the prasugrel.

In the context of the present application "unbound water" may be in particular determined by thermogravimetry according to procedures known to a skilled person. According to a preferred embodiment, thermogravimetry using an isothermal heating (3h/70°C) can be carried out. According to a further preferred embodiment, an isothermal heating program (3h/70°C) can be carried out for a sample of 10 mg at a flow rate of nitrogen of 40 ml / minute in a Mettler Toledo TGA/DSC 1 according to the manual instructions provided for Mettler Toledo TGA/DSC 1 (Mettler-Toledo GmbH, Giessen, DE). Most preferred, the method as disclosed in Section K) "The content of unbound water of formulations according to the present invention" (Temperature program: isothermal 3h/70°C) may be used.

"Unbound solvent" may be in the context of the present application in particular determined by thermogravimetry according to procedures known to a skilled person.

In a further embodiment of the invention, the total amount of water of the composition may be chosen to be below 4 weight%, preferably below 3.5 weight% and more preferably below 3 weight% of the total weight of the composition in order to avoid undesired chemical degradation of the prasugrel.

In order to optimize chemical stability of prasugrel in solid composition it has been found out that the mass ratio beetween xylitol for direct compression such as (eg. direct compressible xylitol, granulated and or spray dryed xylitol, or coprocessed xylitol) and low moisture microcrystalline cellulose in the solid compositions according to present invention should be from 10:1 to 1:1.5, preferably from 5:1 to 1:1.

During the process and storage of solid composition comprising prasugrel of the present invention no change in polymorphic form of prasugrel has been observed according to X-ray powder diffraction patterns.

The dosage of the compound of said formula (1) or a pharmacologically acceptable salt thereof as active ingredient of the solid medicinal preparation of this invention may vary in function of the conditions such as the activity of the medicine, the disease, age and body weight of the patient. The dose in case of oral application for an adult is normally and daily from 0.01 mg (preferably 1 mg) as bottom limit to 200 mg (preferably 100 mg) as top limit. Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister or a bottle. Any pharmaceutical composition may be prepared and stored in a packaging material usually chosen by those of ordinary skill in the art of maintaining stability of active drugs. Decreased gas permeability of primary packaging material is preferred in order to diminish moisture sorption by prasugrel containing pharmaceutical composition to avoid any changes in drug stability. Specially preferred is packaging material having decreased permeability for oxygen and/or water vapor (moisture). Decreased permeability for moisture means that the water permeability measured according to DIN 53122 is below 0.5 g/(m²*day). Decreased gas permeability means gas, especially oxygen, permeability of less than 0.1cm³*day and is measured according to DIN 53380. Low moisture permeable primary packaging materials such as polychloro-3-fluoroethylene homopolymer/PVC laminate can be used with the thickness in the range 270 µm to 360 µm. In case of Alu/Alu blisters a multilayer laminate with the total thickness in the range 100 µm to 155 µm and thickness of aluminium foil layer in the range 5 µm to 100 µm, preferably 7 µm to 60 µm, can be used. Optionally, dosage forms containing prasugrel or its pharmaceutically acceptable salt can be packed into primary packaging with desiccant. Desiccant, containing material having high capacity for moisture sorption such as silica, molecular sieve, zeolite, can be placed inside the packaging unit together with prasugrel dosage units such as tablets or capsules and/or in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers.

For example, as packaging material blisters, strips, glass bottles, containers made of polymeric materials with suitable closure systems may be used. Packaging can be performed under normal atmosphere or optionally under an atmosphere having a reduced oxygen concentration, preferably in an inert atmosphere or under reduced relative humidity such as 40% RH, preferably 35% RH and most preferably below 30% RH. Reduced oxygen concentration means that the concentration of oxygen in the gas surrounding the solid composition in the primary packaging is below 10 vol/vol%, preferably below 5 vol/vol% and most preferably below 2 vol/vol%.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidity-activated oxygen absorbers, ultraviolet-radiation-activated absorbers, radiation-activated absorbers, microwave-radiation-activated absorbers, absorbers activated by a combination of activation processes or absorbers without necessity of activation. Examples of commercially available absorbers are Ageless™ (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

The invention also provides a stabilized package of prasugrel which is provided with a space for trapping and disposal of free oxygen. Moreover, if the active compounds of the present composition are exhibited to a reduced oxygen partial pressure, the formulation is preferably enclosed in a substantially gas exchange non-pernieable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister.

The pharmaceutical preparation of the present invention may be in any form such as, for example, tablets, orally dispersible pharmaceutical formulations, capsules, pellets, granulate, etc., suitable to be stored in a gas exchange non-permeable package. The dosage form is preferably suitable for oral application. Preferably, the dosage form for oral application is a tablet, most preferably film-coated tablet.

Due to low solubility of prasugrel it is important to control the particle size and particle size distribution of prasugrel. In the formulation of the
present invention, the average particle size of prasugrel or its pharmaceutically acceptable salts is
in the range of 0.2 µm to 150 µm, as measured by laser light
diffraction.

The maximum primary particle diameter is preferably 500 µm, more preferably 300 µm as determined by microscopic analysis. To achieve required dissolution of prasugrel from the solid composition containing prasugrel in the form of free base the average particle size has to be preferably below 150 µm and more preferably below 50 µm. Average particle size of prasugrel is most preferably in the range of 0.2 µm to 20 µm. The average particle size is determined by laser diffraction method such as by Malvern instrument

The term "average particle size" as used herein refers to the volume mean particle diameter. The term "maximum primary particle diameter" as used herein refers to a diameter measured as the longest distance of a singular particle.

Any ingredient can be incorporated into the composition of the present invention into intragranular phase (i.e. into granulate), extragranular phase (i.e. as addition to the granulate) or can be incorporated partially into intra- and extragranular phase.

Optionally, cores/tablets can be coated with conventional materials used for film coating, i.e. as described in Pharmaceutical Coating Technology (G. Cole (ed.), 1995). Film coating formulations usually contain the following components:
- polymer(s),
- plasticizer(s),
- colourant(s)/opacifier(s),
- vehicle(s).

In film coating a dispesion of minor quantities of flavors, surfactants and waxes can be used. The majority of the polymers used in film coating are either cellulose derivatives, such as cellulose ethers, or acrylic polymers and copolymers. Occasionally encountered are high molecular weight polyethylene glycols, polyvinyl pyrrolidone, polyvinyl alcohol and waxy materials. Their function usually is to prevent bad mouth feel and/or taste and in some cases degradation, e.g. oxidation of the active ingredients and/or pharmaceutical additives used. Typical cellulose ethers which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further be modified to enhance swelling and permeability by the incorporation of materials such as water soluble cellulose ethers and starches in order to ensure complete disintegration/dissolution of the film.

The commonly used plasticizers can be categorized into three groups:
- polyols (glycerol, propylene glycol, macrogols),
- organic esters (phthalate esters, dibutyl sebacetate, citrate esters, triacetin),
- oils/glycerides (castor oil, acetylated monoglycerides, fractionated coconut oil).

Colourants/opacifiers are classified into several groups:
- organic dyes and their lakes,
- inorganic colours,
- natural colours.

Optionally soluble diluents having solubility in water of more than 1 g in 15 ml of water at 25°C, such as xylitol, maltitol, sorbitol, izomalt, lactitol, dextrose can optionally be used in film coating to enhance the dissolution of the coating.

Different materials from each group can also be combined in defined ratios. Film coating suspensions can also be used as ready-to-make preparations which are available on the market.

Film coating dispersions can be prepared by using different solvents, protic or aprotic, like water or organic solvents such as alcohols (e.g. methanol, ethanol, isopropanol), ketones (e.g. acetone), and mixtures thereof. The preferred solvent is water.

A composition of coating suspension (calculated on dry material) which comprises:
- 1-99%, preferably 1-95% by weight of polymer,
- 1-50%, preferably 1-40% by weight of plasticizer,
- 0.1-20%, preferably 0.1-10% by weight of colourant/opacifier,
is particularly preferred.

Further active agents can be incorporated in pharmaceutical composition according present invention, where the weight ratio of prasugrel to second active agent is in the range 1:1 to 1:100, preferably 1:2 to 1:50. Further active agents can be from the group of antiplatelet agents such as acetylsalicylic acid, clopidrogrel, statins (e.g. atorvastatin, simvastatin, lovastatin, pitavastatin, fluvastatin or rosuvastatin), sartans (e.g. losartan, valsartan, candesartan, olmesartan, irbesartan, telmisartan or eprosartan), prazoles (e.g. lansoprazole, omeprazole, esomeprazole, rabeprazole), direct thrombine inhibitors like e.g. rivaroxabane or inhibitors of factor Xa like e.g. dabigatran.

In one of the most preferred embodiments micronized prasugrel base (with average particle size between 3 and 6 microns) was mixed in a tumbling blender with croscarmellose sodium. Afterwards the mixture of prasugrel and croscarmellose sodium was mixed in a tumbling blender with previously prepared mixture of xylitol, microcrystalline cellulose, fumaric acid and/or sodium lauryl sulfate. Finally magnesium stearate was admixed to the obtained mixture of powders and the obtained compression mixture was then compressed into tablets. Preferably, tablets are film coated using a standard water based film coating based on water soluble polymers and optinal other excipients.Dry component of coating dispersion according to present invention can contain 10-80 wt% water soluble polymer such as cellulose ethers like hypromellose, hyprolose, hydroxyethylcellulose, having viscosity of 2% aqueous solution at 25°C below 20 mPas, preferably below 15mPas, copovidone, povidene, graft copolymer of polyvinyl alcohol and polyethyleneglicole, methacrylic acid copolymers, aminoalkyl methacrylate copolymer, 0-60 wt% soluble diluents such as xylitol, maltitol, sorbitol, izomalt, lactitol, dextrose, 0-30 wt% pigment and/or soluble colourant like titanium dioxide or iron oxide, 0 - 20 wt% plasticizer such as polyethylenglycole having average molecular weight of less than 10,000, propileneglycole, triethylcitrate, triacetine, dibutilsebacate, 0-10 wt% surfactant like sodium dodecyl sulphate, polysorbate, poloxamere

In a second most preferred embodiment the micronized prasugrel base (with average particle size between 3 and 6 microns) was first mixed with half of disintegrant (croscarmellose sodium). Afterwards half of diluents selected from microcrystalline cellulose and xylitol) and the whole amount of sodium lauryl sulfate, as well as the whole amount of fumaric acid were added to the mixture of prasugrel and croscarmellose sodium and mixed in a tumbling blender. Afterwards the ingredients were added and blended in a tumbling blender also the second half of croscarmellose sodium, microcrystalline cellulose and xylitol were added. Separately the whole amount of magnesium stearate were admixed and the obtained compression mixture was compressed into tablets.
Preferably, tablets are film coated using a standard water based film coating based on water soluble polymers and optinal other excipients.Dry component of coating dispersion according to present invention can contain 10-80 wt% water soluble polymer such as cellulose ethers like hypromellose, hyprolose, hydroxyethylcellulose, having viscosity of 2% aqueous solution at 25°C below 20 mPas, preferably below 15mPas, copovidone, povidene, graft copolymer of polyvinyl alcohol and polyethyleneglicole, methacrylic acid copolymers, aminoalkyl methacrylate copolymer, 0-60 wt% soluble diluents such as xylitol, maltitol, sorbitol, izomalt, lactitol, dextrose, 0-30 wt% pigment and/or soluble colourant like titanium dioxide or iron oxide, 0 - 20 wt% plasticizer such as polyethylenglycole having average molecular weight of less than 10,000, propileneglycole, triethylcitrate, triacetine, dibutilsebacate, 0-10 wt% surfactant like sodium dodecyl sulphate, polysorbate, poloxamere

In the last most preferred embodiment micronized prasugrel base , a part of croscarmellose sodium and all of the following excipients: poloxamer (e.g. poloxamer 188), powdered cellulose, fumaric acid, coloidal silicon dioxide and talc were mixed in a high shear mixer equipped with a double jacked product bown and heated to aproximately the melting temperature of the binder while mixing. The mixing was continued until granulate was obtained, the granulate was cooled to room temperature and sieved with an oscilating sieve. The granulate obtained was mixed with xylitol, microcrystalline cellulose and the remaining croscarmellose sodium. Afterwards magnesium stearate was admixed to the obtained mixture, and the resulted compression mixture was compressed into tablets.
Preferably, tablets are film coated using a standard water based film coating based on water soluble polymers and optinal other excipients.Dry component of coating dispersion according to present invention can contain 10-80 wt% water soluble polymer such as cellulose ethers like hypromellose, hyprolose, hydroxyethylcellulose, having viscosity of 2% aqueous solution at 25°C below 20 mPas, preferably below 15mPas, copovidone, povidene, graft copolymer of polyvinyl alcohol and polyethyleneglicole, methacrylic acid copolymers, aminoalkyl methacrylate copolymer, 0-60 wt% soluble diluents such as xylitol, maltitol, sorbitol, izomalt, lactitol, dextrose, 0-30 wt% pigment and/or soluble colourant like titanium dioxide or iron oxide, 0 - 20 wt% plasticizer such as polyethylenglycole having average molecular weight of less than 10,000, propileneglycole, triethylcitrate, triacetine, dibutilsebacate, 0-10 wt% surfactant like sodium dodecyl sulphate, polysorbate, poloxamer.

The invention is illustrated by the following examples:

### EXAMPLES

### A.) Preparation of tablets comprising 10 mg prasugrel base by direct compression (Reference)

As stated in tables 2, 3 and 4 prasugrel base was mixed in a tumbling blender with the following ingredients: one or more diluents, (selected from cellulose, microcrystalline, directly compressible xylitol, silicified microcrystalline cellulose), disintegrant (selected from pregelatinized starch, croscarmellose sodium, sodium starch glycolate, crospovidone), glidant (colloidal silicon dioxide) optionally sodium lauryl sulfate (examples 6A, 11A, 16A, 21A) and in examples 7A, 12A, 13A, 14A, 17A, 21A also citric acid, anhydrous BHT (butylated hydroxytoluene) and BHA (butylated hydroxyanisole).

Afterwards a lubricant selected from magnesium stearate, stearic acid, talc, sodium stearyl fumarate, castor oil, hydrogenated, was admixed to the previously prepared mixture of powders.

The obtained compression mixture was then compressed into tablets.

Preferably, tablets are film coated using a standard water based film coating using HPMC or PVA. Example of water based HPMC film coating composition is as follows:

| *Coating layer* | |
|---|---|
| Hypromellose 6 cp | 5.60 |
| Titanium dioxide | 1.50 |
| Talc | 0.50 |
| Propylene glycol | 0.40 |

The same procedure was applied in Examples 1A-21A.

### B.) Preparation of tablets comprising 10 mg prasugrel base by compaction (Reference)

With the same compositions as in Examples 7A, 12A, 14A, 18A and 21A, the prasugrel base was mixed with approximately half of diluents (selected from cellulose, microcrystalline, directly compressible xylitol, silicified microcrystalline cellulose), half of disintegrant (crospovidone or pregelatinized starch) and one third of lubricant (talc or magnesium stearate), optionally the whole amount of sodium lauryl sulfate (examples 12B, 14B, 21B), and in examples 7B, 12B, 14B and 21B also the whole amount of citric acid, anhydrous BHT (butylated hydroxytoluene) and BHA (butylated hydroxyanisole). The mixture was compacted on a Fitzpatrick chilsonator roller compactor with a built-in mill and sieved.

Afterwards the following ingredients were added and blended in a tumbling blender: half of selected diluents, half of disintegrant, and in examples 7B, 12B, 14B and 21B the whole amount of colloidal silicon dioxide.

Separately the remaining two thirds of talc or magnesium stearate were admixed and the obtained compression mixture was compressed into tablets.

Preferably, tablets are film coated using a standard water based film coating using HPMC or PVA. Example of water based HPMC film coating composition is as follows:

| *Coating layer* | |
|---|---|
| Hypromellose 6 cp | 5.60 |
| Titanium dioxide | 1.50 |
| Talc | 0.50 |
| Propylene glycol | 0.40 |

The same procedure was applied in Examples 7B, 12B, 14B, 18B and 21B.

**Table 2: Composition of the tablets of Examples 1 to 7.**

| Ingredients (amounts in mg per one tablet) | Ex. 1 /mg | Ex. 2 /mg | Ex. 3 /mg | Ex. 4 /mg | Ex. 5 /mg | Ex. 6 /mg | Ex. 7 /mg |
|---|---|---|---|---|---|---|---|
| Prasugrel | 10.00 | 10.00 | 10.00 | 10.00 | 10.00 | 10.00 | 10.00 |
| Pregelatinized starch* | 45.00 | 45.00 | 45.00 | 45.00 | 45.00 | 45.00 | 45.00 |
| Microcrystalline cellulose** | 209.60 | 206.90 | 201.50 | 206.90 | 201.50 | 205.60 | 207.70 |
| Colloidal silicon dioxide*** | 2.70 | 2.70 | 2.70 | 2.70 | 2.70 | 2.70 | 2.70 |
| Magnesium stearate | 2.70 | | | | | 2.70 | 2.70 |
| Stearic acid | | 5.40 | | | | | |
| Talc | | | 10.80 | | | | |
| Sodium stearyl fumarate | | | | 5.40 | | | |
| Castor oil. hydrogenated | | | | | 10.80 | | |
| Sodium lauryl sulfate | | | | | | 4.00 | |
| Citric acid. anhydrous | | | | | | | 1.80 |
| BHT (Butylated hydroxytoluene) | | | | | | | 0.05 |
| BHA (Butylated hydroxyanisole) | | | | | | | 0.05 |
| Total | 270.00 | 270.00 | 270.00 | 270.00 | 270.00 | 270.00 | 270.00 |

| | | | | | | | |
|---|---|---|---|---|---|---|---|
| * commercially available as Starch 1500 LM ** commercially available as Avicel PH 200 LM or AVICEL PH 112 *** commercially available as Aerosil 200D or Aerosil R972 | | | | | | | |

**Table 3: Composition of the tablets of Examples 8 to 14.**

| Ingredients (amounts in mg per one tablet) | Ex. 8 /mg | Ex. 9 /mg | Ex. 10 /mg | Ex. 11 /mg | Ex. 12 /mg | Ex. 13 /mg | Ex. 14 /mg |
|---|---|---|---|---|---|---|---|
| Prasugrel | 10.00 | 10.00 | 10.00 | 10.00 | 10.00 | 10.00 | 10.00 |
| Xylitab^{®} 100^{a} | 127.30 | 127.30 | 127.30 | 127.30 | 127.30 | | 166.40 |
| Xylitab^{®} 200^{b} | | | | | | 127.30 | |
| Microcrystalline cellulose^{c} | 116.50 | 116.50 | 116.50 | 112.50 | 114.60 | 114.60 | 75.50 |
| Croscarmellose sodium^{d} | 10.80 | | | | | | |
| Sodium starch glycolate^{e} | | 10.80 | | | | | |
| Crospovidone^{f} | | | 10.80 | 10.80 | 10.80 | 10.80 | 10.80 |
| Sodium lauryl sulfate | | | | 4.00 | | | |
| Colloidal silicon dioxide | 2.70 | 2.70 | 2.70 | 2.70 | 2.70 | 2.70 | 2.70 |
| Magnesium stearate | 2.70 | 2.70 | 2.70 | 2.70 | 2.70 | 2.70 | 2.70 |
| Citric acid. anhydrous | | | | | 1.80 | 1.80 | 1.80 |
| BHT (Butylated hydroxytoluene) | | | | | 0.05 | 0.05 | 0.05 |
| BHA (Butylated hydroxyanisole) | | | | | 0.05 | 0.05 | 0.05 |
| Total | 270.00 | 270.00 | 270.00 | 270.00 | 270.00 | 270.00 | 270.00 |

| | | | | | | | |
|---|---|---|---|---|---|---|---|
| ^{a} commercially available directly compressible xylitol (binder: max. 3.5 % polydextrose) ^{b} commercially available directly compressible xylitol (binder: max. 2 % sodium carboxymethylcellulose) ^{c} commercially available Avicel PH 200 LM or AVICEL PH 112 ^{d} commercially available as Ac-Di-Sol ^{e} commercially available as Primojel ^{f} commercially available as Kollidon CL | | | | | | | |

**Table 4: Composition of the tablets of Examples 15 to 21.**

| Ingredients (amounts in mg per one tablet) | Ex. 15 /mg | Ex. 16 /mg | Ex. 17 /mg | Ex. 18 /mg | Ex. 19 /mg | Ex. 20 /mg | Ex. 21 /mg |
|---|---|---|---|---|---|---|---|
| Prasugrel | 10.00 | 10.00 | 10,00 | 10.00 | 10.00 | 10.00 | 10.00 |
| Silicified microcrystalline cellulose⁺ | 246.50 | 242.50 | 244.60 | 204.20 | 85.00 | 85.00 | 115.00 |
| Xylitab^{®} 100⁺⁺ | | | | | 124.60 | 116.50 | 114.80 |
| Pregelatinized starch⁺⁺⁺ | | | | 45.00 | 45.00 | 45.00 | |
| Crospovidone⁺⁺⁺⁺ | 10.80 | 10.80 | 10.80 | | | | 10.80 |
| Colloidal silicon dioxide⁺⁺⁺⁺⁺ | | | | | 2.70 | 2.70 | 2.70 |
| Magnesium stearate | 2.70 | 2.70 | 2.70 | | 2.70 | | |
| Talc | | | | 10.80 | | 10.80 | 10.80 |
| Sodium lauryl sulfate | | 4.00 | | | | | 4.00 |
| Citric acid, anhydrous | | | 1.80 | | | | 1.80 |
| BHT (Butylated hydroxytoluene) | | | 0.05 | | | | 0.05 |
| BHA (Butylated hydroxyanisole) | | | 0.05 | | | | 0.05 |
| Total | 270.00 | 270.00 | 270.00 | 270.00 | 270.00 | 270.00 | 270.00 |

| | | | | | | | |
|---|---|---|---|---|---|---|---|
| ⁺ commercially available as Prosolv SMCC 90 or Prosolv SMCC 90 LM ⁺⁺ commercially available directly compressible xylitol (binder: max. 3.5 % polydextrose) ⁺⁺⁺ commercially available Starch 1500 LM ⁺⁺⁺⁺ commercially available Kollidon CL ⁺⁺⁺⁺⁺ commercially available Aerosil 200D or Aerosil R972 | | | | | | | |

### C.) Preparation of tablets comprising 5 mg prasugrel base by direct compression (Reference)

As stated in table 5 prasugrel base was mixed in a tumbling blender with the following ingredients: one ore more diluents (selected from cellulose, microcrystalline, directly compressible xylitol, silicified microcrystalline cellulose), disintegrant (crospovidone), glidant (colloidal silicon dioxide) optionally sodium lauryl sulfate (examples 22A-28A) and in examples 22A-25A and 27A-28A also citric acid, anhydrous BHT (butylated hydroxytoluene) and BHA (butylated hydroxyanisole).

Afterwards a lubricant selected from magnesium stearate, castor oil, hydrogenated, was admixed to the previously prepared mixture of powders.

The obtained compression mixture was then compressed into tablets.

Preferably, tablets are film coated using a standard water based film coating using HPMC or PVA. Example of water based HPMC film coating composition is as follows:

| *Coating layer* | |
|---|---|
| Hypromellose 6 cp | 5.60 |
| Titanium dioxide | 1.50 |
| Talc | 0.50 |
| Propylene glycol | 0.40 |

The same procedure was applied in Examples 22A-28A.

### D.) Preparation of tablets comprising 5 mg prasugrel base by compaction (Reference)

With the same compositions as in Examples 23A, 24A and 28A, the prasugrel base was mixed with approximately half of diluents (selected from microcrystalline cellulose, directly compressible xylitol, silicified microcrystalline cellulose), half of disintegrant (crospovidone or pregelatinized starch) and one third of talc, and the whole amount of sodium lauryl sulfate, citric acid, anhydrous BHT (butylated hydroxytoluene) and BHA (butylated hydroxyanisole). The mixture was compacted on a Fitzpatrick chilsonator roller compactor with a built-in mill and sieved.

Afterwards the following ingredients were added and blended in a tumbling blender: half of selected diluents, half of disintegrant, and in examples 23B and 24B the whole amount of colloidal silicon dioxide.

Separately the remaining two thirds of talc were admixed and the obtained compression mixture was compressed into tablets.

Preferably, tablets are film coated using a standard water based film coating using HPMC or PVA. Example of water based HPMC film coating composition is as follows:

| *Coating layer* | |
|---|---|
| Hypromellose 6 cp | 5.60 |
| Titanium dioxide | 1.50 |
| Talc | 0.50 |
| Propylene glycol | 0.40 |

The same procedure was applied in Examples 23B, 24 B and 28B.

**Table 5: Composition of the tablets of Examples 22 to 28.**

| Ingredients (amounts in mg per one tablet) | Ex. 22 /mg | Ex. 23 /mg | Ex. 24 /mg | Ex. 25 /mg | Ex. 26 /mg | Ex. 27 /mg | Ex. 28 /mg |
|---|---|---|---|---|---|---|---|
| Prasugrel | 5.00 | 5.00 | 5.00 | 5.00 | 5.00 | 5.00 | 5.00 |
| Pregelatinized starch* | 45.00 | 45.00 | | | 45.00 | 45.00 | 45.00 |
| Microcrystalline cellulose** | 210.70 | 202.60 | 117.60 | 78.50 | | | |
| Xylitab^{®} 100*** | | | 119.20 | 166.40 | 130.3 | 128.40 | |
| Silicified microcrystalline cellulose**** | | | | | 85.00 | 85.00 | 205.30 |
| Crospovidone***** | | | 10.80 | 10.80 | | | |
| Colloidal silicon dioxide****** | 2.70 | 2.70 | 2.70 | 2.70 | | | |
| Magnesium stearate | 2.70 | | | 2.70 | 2.70 | 2.70 | |
| Talc | | 10.80 | 10.80 | | | | 10.80 |
| Sodium lauryl sulfate | 2.00 | 2.00 | 2.00 | 2.00 | 2.00 | 2.00 | 2.00 |
| Citric acid anhydrous | 1.80 | 1.80 | 1.80 | 1.80 | | 1.80 | 1.80 |
| BHT (Butylated hydroxytoluene) | 0.05 | 0.05 | 0.05 | 0.05 | | 0.05 | 0.05 |
| BHA (Butylated hydroxyanisole) | 0.05 | 0.05 | 0.05 | 0.05 | | 0.05 | 0.05 |
| Total | 270.00 | 270.00 | 270.00 | 270.00 | 270.00 | 270.00 | 270.00 |

| | | | | | | | |
|---|---|---|---|---|---|---|---|
| commercially available as Starch 1500 LM ** commercially available as Avicel PH 200 LM or AVICEL PH 112 *** commercially available directly compressible xylitol (binder: max. 3.5 % polydextrose) **** commercially available as Prosolv SMCC 90 or Prosolv SMCC 90 LM ***** commercially available as Kollidon CL ****** commercially available as Aerosil 200D or Aerosil R972 | | | | | | | |

### E.) Preparation of granulates and tablets comprising prasugrel base 5 mg and 10 mg by hot melt granulation (Reference)

Prasugrel or its salt is mixed with other components except binder (*) and warmed to 40 °C in case of example 29 or 60°C in case of examples 30-32 by heated double jacked product container of Collete Gral 10 l mixer granulator. Melted binder is added to the powder during mixing. The mass is mixed until granulate is obtained. The obtained granulate is cooled down to 25°C and is if needed ground and sieved.

Examples 33 and 34: all components were transferred to the product container of Collete Gral 10 l mixer granulator equipped with heating/cooling jacked and heated to the temperature 5°C above melting point of binder. Mixing was continued until granulate was formed. The obtained granulate was cooled to 25°C and is if needed ground and sieved.

**Table 6: Composition of the granulate obtained by hot melt granulation of**

| Ingredients (amounts in %) | Ex. 29 /% w/w | Ex. 30 /% w/w | Ex. 31 /% w/w | Ex. 32 /% w/w | Ex. 33 /% w/w | Ex. 34 /% w/w |
|---|---|---|---|---|---|---|
| Prasugrel | 30.0 | 43.5 | 25.0 | | 43.5 | |
| Prasugrel HCl | | | | 50.0 | | 50.0 |
| Microcrystalline cellulose low | 51.9 | 20.0 | | 30.0 | 15.0 | 29.0 |
| moisture | | | 45.0 | | | |
| Gelucire 44/14* | 13.0 | | | 7.0 | | 5.0 |
| Macrogol 6000* | | 15.0 | 16.0 | | 20.0 | |
| Stearic acid | | | | 7.0 | | 10.0 |
| Crospovidone | | 5.0 | | | 5.0 | |
| Citric acid | 5.0 | 3.5 | 5.0 | 1.0 | 4.5 | 1.0 |
| Colloidal silicon dioxide | | 0.5 | 0.5 | | 0.5 | |
| Talc | | 6.5 | 7.5 | 2.0 | 7.0 | 2.0 |
| Sodium lauryl sulfate | | 5.0 | | 2.0 | 3.5 | 2.0 |
| BHT (Butylated hydroxytoluene) | 0.1 | 0.5 | 1.0 | | | |
| BHA (Butylated hydroxyanisole) | | 0.5 | | 1.0 | 1.0 | 1.0 |
| Total | 100.0 | 100.0 | 100.0 | 100.0 | 100.0 | 100.0 |

### Preparation of prasugrel tablets from granulate obtained by hot melt granulation (Reference or Invention (Tables 8 and 9))

The granulate obtained in examples 29-31 was mixed with Avicel PH200 LM, castor oil, hydrogenated and Aerosil, and compressed into tablets.

**Table 7: Composition of tablets obtained via hot melt granulation**

| Ingredients (amounts in mg per one tablet) | Ex. 35 /mg/tbl. | Ex. 36 /mg/tbl. | Ex. 37 /mg/tbl. |
|---|---|---|---|
| | | | |
| Granulate from example 31 | 40.0 | | |
| Granulate from example 30 | | 11.5 | |
| Granulate from example 29 | | | 33.3 |
| Avicel PH 200 low moisture | 137.8 | 94.9 | |
| Crospovidone | 10.0 | 6.0 | 10.0 |
| Citric acid | 2.0 | 1.5 | 10.0 |
| Colloidal silicon dioxide | 2.0 | 1.5 | 2.0 |
| Prosolv SMCC 90LM | | | 120.0 |
| Xylitab 100 | | | 65.2 |
| BHT (Butylated hydroxytoluene) | 0.2 | 0.1 | |
| Castor oil, hydrogenated | 8.0 | 4.5 | 8.0 |
| Magnesium stearate | | | 1.5 |
| Total cores | 200.0 | 120.0 | 250.0 |
| Film coating: | | | |
| Hypromellose | 5,60 | | |
| Opadray AMB | | | 7.5 |
| Kollicoat Protect | | 5.0 | |
| talc | 0,50 | 1.5 | |
| Titanium dioxide | 1,50 | 1.0 | |
| Propylene glycol | 0,40 | | |
| Iron oxide red | | 0.5 | |

**Table 8: Composition of granulate obtained by hot melt granulation**

| | Composition of granulate (mg/unit) | | | | | | |
|---|---|---|---|---|---|---|---|
| Example | **38a** | **39a** | **40a** | **41a** | **42a** | **43a** | **44a** |
| Components (mg) | | | | | | | |
| Prasugrel | 10.0 | 10.0 | 10.0 | 10.0 | 10.0 | 10.0 | 10.0 |
| Polyethylenglycol 6000 | 10.0 | 18.0 | | | | | |
| Poloxamer 188 | | | 10.0 | 10.0 | 10.0 | 20.0 | 20.0 |
| Microcrystalline cellulose low moisture | 30.0 | | 34.8 | | | | |
| Powdered cellulose | 30.0 | 79.6 | | 34.8 | | 79.6 | 79.6 |
| Xylitol | | | | | 34.8 | | |
| Crospovidone | 3.6 | | | | | 4.8 | |
| Croscarmellose sodium | | 4.8 | 2.4 | 2.4 | 2.4 | | 4.8 |
| Sodium lauryl sulfate | 2.0 | 2.0 | | | | | |
| Colloidal silicon dioxide | 1.6 | 2.0 | 1.0 | 1.0 | 1.0 | 2.0 | 2.0 |
| Talc | 2.8 | 3.6 | 1.8 | 1.8 | 1.8 | 3.6 | 3.6 |
| Weight of intragranular phase | 90.0 | 120.0 | 60.0 | 60.0 | 60.0 | 120.0 | 120.0 |

Granulation process for Example (**38a**): all components except polyethyleneglycol 6000 are mixed in the laboratory high share mixer Collette Gral 10 equipped with double jacked product bowl. Melted binder (polyethyleneglycol 6000) was sprayed onto the powder mixture while mixing. The obtained granulate was cooled to approx. 20°C and sieved with oscilating sieve.

Granulation procedure for Examples (**39a**)-(**44a**): all components are mixed in the laboratory high shear mixer Collette Gral 10 equipped with double jacked product bowl and heated to approx. melting temperature of the binder while mixing. The mixing continued until granulate is obtained. The obtained mass was cooled to approx. 20°C and sieved with oscilating sieve. Average (mean) particle size of active ingredient in all experiments in table 8 was app. 4 microns.

**Table 9: Composition of tablets with granulate (Ex.38-44) from hot melt granulation**

| | Compression mixture | | | | | | |
|---|---|---|---|---|---|---|---|
| Example | **38b** | **39b** | **40b** | **41b** | **42b** | **43b** | **44b** |
| Granulate | **38a** | **39a** | **40a** | **41a** | **42a** | **43a** | **44a** |
| Components (mg) | | | | | | | |
| Granulate | 90.0 | 120.0 | 60.0 | 60.0 | 60.0 | 120.0 | 120.0 |
| Directly compressible xylitol | 39.0 | 42.0 | 82.0 | 82.0 | 82.0 | 20.0 | 20.0 |
| Microcrystalline cellulose | 39.0 | 21.0 | 41.0 | 41.0 | 41.0 | 40.0 | 40.0 |
| Croscarmellose sodium | 10.0 | 15.0 | 15.0 | 15.0 | 15.0 | 8.0 | |
| Crospovidone | | | | | | | 8.0 |
| Magnesium stearate | 2.0 | 2.0 | 2.0 | 2.0 | 2.0 | 2.0 | 2.0 |
| Total mass of tbl. | 180.0 | 200.0 | 200.0 | 200.0 | 200.0 | 190.0 | 190.0 |

The granulate obtained in examples 38a-44a was mixed with directly compressible xylitol, microcrystalline cellulose and croscarmellose sodium or crospovidone. Afterwards magnesium stearate was admixed to the previously prepared mixture, and the obtained compression mixture was compressed into tablets.

Preferably, tablets are film coated.
**Example 41c:** Coating of tablets: Tablets from example 41b were coated with HPMC dispersion containing 6wt% hypromellose, 8wt% lactose monohydrate, 4.4wt% titanium dioxide, 1.6 % triacetin and 80wt% water.
**Example 41d:** Coating of tablets: Tablets from example 41b were coated with Kollicoat Protect dispersion containing 12wt% Kollicoat Protect, 5wt% talc, 3wt% titanium dioxide and 80wt% water.
**Example 41e:** Coating of tablets: Tablets from example 41b were coated with HPC dispersion containing 4.5wt% hydroxypropylcellulose, 3wt% lactose, 1.25wt% titanium dioxide, 1.25wt% polyethylenglycol 6000, 2wt% water, 44wt% absolute ethanol and 44wt% isopropyl alcohol.

### F.) Preparation of tablets comprising micronized or co-micronized prasugrel base by direct compression (Invention)

Milled/Micronized prasugrel base: Prasugrel base was milled and grinded with mechanical mill to micron size or micronized in a fluid jet mill to micron size. Mechanical milling could be done in mechanical mill such as Alpina UPZ 100 mill (Hosokawa Micron LTD.) with rotor speed from 2000- 10 000 rpm. Micronization could be done with fluid jet mill such as Jetmill MC 50 (Jet Pharma S.A.) under a pressure on venturi nozzle of 3-11 bars and pressure in milling chamber of 3-10 bar with feed rate from 2- 30 g/min.

Co-micronized prasugrel base: Prasugrel base was mixed with one or more suitable pharmaceutical additives (selected from crospovidone, croscarmellose sodium, pregelatinized starch, sodium starch glycolate, carboxymethyl cellulose sodium, microcrystalline cellulose, disaccharides, e.g. sucrose etc., polyols, e.g. xylitol, sorbitol etc., surfactants, e.g. sodium lauryl sulfate etc., colloidal silicon dioxide, water-soluble cyclodextrines, organic acids, e.g. L-ascorbic acid, (-)-(2S,3S)-tartaric acid etc.) and co-micronized with fluid jet mill such as Jetnill MC 50 (Jet Pharma S.A.) under a pressure on venturi nozzle of 3-11 bars and pressure in milling chamber of 3-10 bar or milled with mechanical mill such as Alpina UPZ 100 mill (Hosokawa Micron LTD.) with rotor speed from 2000- 10 000 rpm.

As stated in table 10 micronized prasugrel base and in table 11 co-micronized prasugrel base was mixed in a tumbling blender with the following ingredients: one or more diluents, (selected from cellulose, microcrystalline, xylitol, silicified microcrystalline cellulose), disintegrant (selected from pregelatinized starch, croscarmellose sodium, sodium starch glycolate, crospovidone), glidant (colloidal silicon dioxide), optionally surfactant (sodium lauryl sulfate) and optionally organic acid (L-ascorbic acid, (-)-(2S,3S)-tartaric acid, adipic acid, pimelic acid, maleic acid, fumaric acid, citric acid, malic acid, glutaric acid, malonic acid, galic acid, ferrulic acid etc.).

Afterwards a lubricant selected from magnesium stearate, stearic acid, talc, sodium stearyl fumarate, castor oil, hydrogenated, was admixed to the previously prepared mixture of powders.

The obtained compression mixture was then compressed into tablets.

**Table 10: Composition of the tablets of Examples 45 to 51.**

| Ingredients (amounts in mg per one tablet) | Ex. 45 /mg | Ex. 46 /mg | Ex. 47 /mg | Ex. 48 /mg | Ex. 49 /mg | Ex. 50 /mg | Ex. 51 /mg |
|---|---|---|---|---|---|---|---|
| Micronized prasugrel base | 5.00 | 10.00 | 60.00 | 10.00 | 10.00 | 10.00 | 10.00 |
| Xylitab^{®} 100^{a} | 120.00 | 116.25 | 202.50 | 112.89 | 116.00 | 111.00 | 126.89 |
| Microcrystalline cellulose low moisture^{b} | 120.00 | 116.25 | 202.50 | 55.00 | 55.00 | 55.00 | 35.00 |
| Croscarmellose sodium^{c} | 17.10 | 17.10 | 34.20 | 15.00 | 15.00 | 16.00 | 20.00 |
| Sodium lauryl sulfate | 1.00 | 2.00 | 12.00 | 2.00 | 2.00 | 5.00 | 3.00 |
| L-ascorbic acid | 1.50 | 3.00 | 18.00 | | | 1.00 | |
| Fumaric acid | | | | 3.11 | | | 3.11 |
| Colloidal silicon dioxide | 2.70 | 2.70 | 5.40 | | | | |
| Magnesium stearate | 2.70 | 2.70 | 5.40 | 2.00 | 2.00 | 2.00 | 2.00 |
| Total | 270.00 | 270.00 | 540.00 | 200.00 | 200.00 | 200.00 | 200.00 |

| | | | | | | | |
|---|---|---|---|---|---|---|---|
| ^{a} commercially available directly compressible xylitol (binder: max. 3.5 % polydextrose) ^{b} commercially available as Avicel PH 200 LM or AVICEL PH 112 ^{c} commercially available as Ac-Di-Sol | | | | | | | |

### Preparation of compression mixture of Ex.45-51:

First of all micronized prasugrel was mixed in a tumbling blender with croscarmellose sodium. Afterwards the mixture of prasugrel and croscarmellose sodium was mixed in a tumbling blender with previously prepared mixture of other ingredients stated in table 10 except magnesium stearate. Finally magnesium stearate was admixed to the previously prepared mixture of powders and the obtained compression mixture was then compressed into tablets.

Average (mean) particle size of active ingredient in Examples 45-47 was 49 µm; in Examples 48-51 was 4 µm; according to laser diffraction method: Pharm. Eur. 2.9.31, Version 6.6., Malvern Mastersizer.

The content of unbound water was determined in examples 46, 48, 49, 50 and 51:

| Example | Content of unbound water |
|---|---|
| Ex. 46 | 3,60 % |
| Ex. 48 | 2,40 % |
| Ex. 49 | 2,60 % |
| Ex. 50 | 2,60 % |
| Ex. 51 | 2,20 % |

Preferably, tablets are film coated.
**Example 51a**: Coating of tablets: Tablets from example 51 were coated with HPMC dispersion containing 6wt% hypromellose, 8wt% lactose monohydrate, 4.4wt% titanium dioxide, 1.6 % triacetin and 80wt% water.
**Example 51b:** Coating of tablets: Tablets from example 51 were coated with Kollicoat Protect dispersion containing 12wt% Kollicoat Protect, 5wt% talc, 3wt% titanium dioxide and 80wt% water.
**Example 51c:** Coating of tablets: Tablets from example 51 were coated with HPC dispersion containing 4.5wt% hydroxypropylcellulose, 3wt% lactose monohydrate, 1.25wt% titanium dioxide, 1.25wt% polyethylenglycol 6000, 2wt% water, 44wt% absolute ethanol and 44wt% isopropanol.

**Table 11: Composition of the tablets of Examples 52 to 58.**

| Ingredients (amounts in mg per one tablet) | Ex. 52 /mg | Ex. 53 /mg | Ex. 54 /mg | Ex. 55 /mg | Ex. 56 /mg | Ex. 57 /mg | Ex. 58 /mg |
|---|---|---|---|---|---|---|---|
| **Co-micronization** | | | | | | | |
| Prasugrel base | 5.00 | 10.00 | 10.00 | 10.00 | 10.00 | 10.00 | 60.00 |
| Crospovidone | 5.00 | 10.00 | 20.00 | | | 10.00 | |
| Xylitol | | | | 50.00 | | | 300.00 |
| Pregelatinized starch^{a} | | | | | 50.00 | | |
| Sodium lauryl sulfate | | | | | | 1.33 | |

| **Additional pharmaceutical additives** | | | | | | | |
|---|---|---|---|---|---|---|---|
| Xylitab^{®} 100^{c} | 119.00 | 113.75 | 101.25 | 25.00 | 30.70 | 113.75 | |
| Microcrystalline cellulose^{b} | 119.00 | 113.75 | 101.25 | 75.00 | 80.70 | 113.75 | 105.00 |
| Croscarmellose sodium^{d} | 14.10 | 12.10 | 2.10 | 11.40 | | 12.10 | 34.20 |
| Sodium lauryl sulfate | 1.00 | 2.00 | 12.00 | 2.00 | 2.00 | 0.67 | 12.00 |
| L-ascorbic acid | 1.50 | 3.00 | 18.00 | 3.00 | 3.00 | 3.00 | 18.00 |
| Colloidal silicon dioxide | 2.70 | 2.70 | 2.70 | 1.80 | 1.80 | 2.70 | 5.40 |
| Magnesium stearate | 2.70 | 2.70 | 2.70 | 1.80 | 1.80 | 2.70 | 5.40 |
| Total | 270.00 | 270.00 | 270.00 | 180.00 | 180.00 | 270.00 | 540.00 |

| | | | | | | | |
|---|---|---|---|---|---|---|---|
| ^{a} commercially available as Starch 1500 LM ^{b} commercially available as Avicel PH 200 LM or AVICEL PH 112 ^{c} commercially available directly compressible xylitol (binder: max. 3.5 % polydextrose) ^{d} commercially available as Ac-Di-Sol | | | | | | | |

### G.) Preparation of tablets comprising prasugrel maleate and prasugrel hemimaleate by direct compression (Reference)

As stated in table 12 prasugrel maleate or prasugrel hemimaleate was mixed in a tumbling blender with the following ingredients: one or more diluents, (selected from cellulose, microcrystalline, xylitol, silicified microcrystalline cellulose), disintegrant (selected from pregelatinized starch, croscarmellose sodium, sodium starch glycolate, crospovidone), glidant (colloidal silicon dioxide), optionally surfactant (sodium lauryl sulfate).and optionally organic acid (L-ascorbic acid, (-)-(2S,3S)-tartaric acid, adipic acid, pimelic acid, maleic acid, fumaric acid, citric acid, malic acid, glutaric acid, malonic acid, galic acid, ferrulic acid etc.) Afterwards a lubricant selected from magnesium stearate, stearic acid, talc, sodium stearyl fumarate, castor oil, hydrogenated, was admixed to the previously prepared mixture of powders.

The obtained compression mixture was then compressed into tablets.

Preferably, tablets are film coated using a standard water based film coating using HPMC or PVA. Example of water based HPMC film coating composition is as follows:

| *Coating layer* | |
|---|---|
| Hypromellose 6 cp | 5.60 |
| Titanium dioxide | 1.50 |
| Talc | 0.50 |
| Propylene glycol | 0.40 |

**Table 12: Composition of the tablets of Examples 59 to 64.**

| Ingredients (amounts in mg per one tablet) | Ex. 59 /mg | Ex. 60 /mg | Ex. 61 /mg | Ex. 62 /mg | Ex. 63 /mg | Ex. 64 /mg |
|---|---|---|---|---|---|---|
| Prasugrel maleate | 6.55 | | 13.11 | | 13.11 | 78.65 |
| Prasugrel hemimaleate | | 5.78 | | 11.55 | | |
| Xylitab^{®} 100^{a} | 119.95 | 120.72 | 112.89 | 114.45 | 84.89 | 306.35 |
| Microcrystalline cellulose^{b} | 55.00 | 55.00 | 55.00 | 55.00 | 84.00 | 155.00 |
| Croscarmellose sodium^{c} | 16.00 | 16.00 | 16.00 | 16.00 | 14.00 | 48.00 |
| L-ascorbic acid | 0.50 | 0.50 | 1.00 | 1.00 | | |
| Colloidal silicon dioxide | | | | | 2.00 | 6.00 |
| Magnesium stearate | 2.00 | 2.00 | 2.00 | 2.00 | 2.00 | 6.00 |
| Total | 200.00 | 200.00 | 200.00 | 200.00 | 200.00 | 600.00 |

| | | | | | | |
|---|---|---|---|---|---|---|
| ^{a} commercially available as directly compressible xylitol (binder: max. 3.5 % polydextrose) ^{b} commercially available as Avicel PH 200 LM or AVICEL PH 112 ^{c} commercially available as Ac-Di-Sol | | | | | | |

The content of unbound water was determined in example 61:

| **Example** | Content of unbound water |
|---|---|
| **Ex. 61** | 2,40 % |

### H.) Preparation of film coated tablets comprising micronized prasugrel base by direct compression, roller compaction and hot melt granulation (Invention)

### H/1: Direct compression

**Table 13: Composition of granulate obtained by direct compression and roller compaction**

| Ingredients (amounts in mg per one tablet) | Ex. 65 | Ex. 66 | Ex. 67 | Ex. 68 | Ex. 69 | Ex. 70 | Ex. 71 |
|---|---|---|---|---|---|---|---|
| Prasugrel base* | 5.00 | 10.00 | 10.00 | 10.00 | 10.00 | 10.00 | 60.00 |
| Granulated or spray dryed xylitol** | 63.45 | 126.90 | | 130.00 | 128.90 | 81.90 | 342.50 |
| Xylitol for direct compression*** | | | 122.45 | | | | |
| Polydextrose | | | 4.45 | | | | |
| Microcrystalline cellulose low moisture **** | 17.50 | 35.00 | 35.00 | 35.00 | 36.00 | 80.00 | 94.90 |
| Croscarmellose sodium | 10.00 | 20.00 | 20.00 | 20.00 | 20.00 | 20.00 | 60.00 |
| Sodium lauryl sulfate | 1.50 | 3.00 | 3.00 | 3.00 | | 3.00 | 18.00 |
| Fumaric acid | 1.55 | 3.10 | 3.10 | | 3.10 | 3.10 | 18.60 |
| Magnesium stearate | 1.00 | 2.00 | 2.00 | 2.00 | 2.00 | 2.00 | 6.00 |
| Total | 100.00 | 200.00 | 200.00 | 200.00 | 200.00 | 200.00 | 600.00 |

| | | | | | | | |
|---|---|---|---|---|---|---|---|
| * particle size distribution of prasugrel base in in all experiments in table 13: average (mean) particle size 4 µm; d₁₀ <1.0 µm; d₅₀ <3.6 µm; d₉₀ <8.4 µm; according to laser diffraction method: Pharm. Eur. 2.9.31, Version 6.6., Malvern Mastersizer, ** commercially available as Xylitab^{®} 100 *** commercially available as Xylitab^{®} 300, Xylisorb^{®} 300 **** commercially available as Avicel PH112, Avicel PH200 LM | | | | | | | |

### Preparation of compression mixture of Ex.65-71:

First of all micronized prasugrel was mixed in a tumbling blender with croscarmellose sodium. Afterwards the mixture of prasugrel and croscarmellose sodium was mixed in a tumbling blender with previously prepared mixture of other ingredients stated in table 13 except magnesium stearate. Finally magnesium stearate was admixed to the previously prepared mixture of powders and the obtained compression mixture was then compressed into tablets.

Preferably, tablets are film coated using a standard water based film coating using HPMC. Example of water based HPMC film coating composition is as follows: HPMC dispersion containing 6wt% hypromellose, 8wt% isomalt, 4.4wt% titanium dioxide, 1.6 % triacetin and 80wt% water.

### H/2: Roller compaction

With the same compositions as stated in table 13 (Examples 65 to 71), the prasugrel base was first of all mixed with half of disintegrant (croscarmellose sodium). Afterwards half of diluents (selected from microcrystalline cellulose low moisture, xylitol) and in all examples in table 13 except Ex. 69 the whole amount of sodium lauryl sulfate, in all examples in table 13 except Ex. 68 the whole amount of fumaric acid and in Ex. 67 the whole amount of polydextrose were added to the mixture of prasugrel and croscarmellose sodium and mixed in a tumbling blender. The prepared mixture was compacted on a Fitzpatrick chilsonator roller compactor with a built-in mill and sieved.

Afterwards the following ingredients were added and blended in a tumbling blender: half of selected diluents and half of disintegrant.

Separately the whole amount of magnesium stearate were admixed and the obtained compression mixture was compressed into tablets.

Preferably, tablets are film coated using a standard water based film coating using HPMC. Example of water based HPMC film coating composition is as follows: HPMC dispersion containing 6wt% hypromellose, 8wtxylitol, 4.4wt% titanium dioxide, 1.6 % triacetin and 80wt% water.

### H.)/3: Hot melt granulation

**Table 14: Composition of granulate obtained by hot melt granulation**

| | Composition of granulate (mg/unit) | | | |
|---|---|---|---|---|
| Example | **72a** | **73a** | **74a** | **75a** |
| Components (mg) | | | | |
| Prasugrel base* | 10.00 | 10.00 | 10.00 | 10.0 |
| Poloxamer 188** | 6.00 | 8.00 | 8.00 | 10.0 |
| Powdered cellulose*** | 38.20 | 38.20 | 36.20 | 34.20 |
| Croscarmellose sodium | 5.00 | 5.00 | 5.00 | 5.00 |
| Fumaric acid | 2.00 | | 2.00 | 2.00 |
| Colloidal silicon dioxide | 1.00 | 1.00 | 1.00 | 1.00 |
| Talc | 1.80 | 1.80 | 1.80 | 1.80 |
| Weight of intragranular phase | 64.00 | 64.00 | 64.00 | 64.00 |

| | | | | |
|---|---|---|---|---|
| * particle size distribution of prasugrel base in in all experiments in table 13: average (mean) particle size 4 µm; d₁₀ <1.0 µm; d₅₀ <3.6 µm; d₉₀ <8.4 µm; according to laser diffraction method: Pharm. Eur. 2.9.31, Version 6.6., Malvern Mastersizer. ** commercially available as Lutrol^{®} F68, Pluronic^{®} F68 *** commercially available as Arbocel^{®} M80 | | | | |

Granulation procedure for Examples **(72a)-(75a)**: all components are mixed in the laboratory high shear mixer Collette Gral 10 equipped with double jacked product bowl and heated to approx. melting temperature of the binder while mixing. The mixing continued until granulate is obtained. The obtained mass was cooled to approx. 20°C and sieved with oscilating sieve.

**Table 15: Composition of tablets with granulate (Ex.72a-75a) from hot melt granulation**

| | Compression mixture | | | | |
|---|---|---|---|---|---|
| Example | **72b** | **73b** | **74b** | **74c** | **75b** |
| Granulate | **72a** | **73a** | **74a** | **74a** | **75a** |
| Components (mg) | | | | | |
| Granulate | 64.00 | 64.00 | 64.00 | 64.00 | 64.00 |
| Granulated or spray dryed xylitol* | 77.00 | 77.00 | 77.00 | | 77.00 |
| Xylitol for direct compression** | | | | 77.00 | |
| Microcrystalline cellulose low moisture*** | 42.00 | 42.00 | 42.00 | 42.00 | 42.00 |
| Croscarmellose sodium | 15.00 | 15.00 | 15.00 | 15.00 | 15.00 |
| Magnesium stearate | 2.00 | 2.00 | 2.00 | 2.00 | 2.00 |
| Total mass of tbl. | 200.00 | 200.00 | 200.00 | 200.00 | 200.00 |

| | | | | | |
|---|---|---|---|---|---|
| *commercially available as Xylitab^{®} 100 ** commercially available as Xylitab^{®} 300, Xylisorb^{®} 300 ***commercially available as Avicel PH112, Avicel PH200 LM | | | | | |

The granulate obtained in examples 72a-75a was mixed with xylitol, microcrystalline cellulose and croscarmellose sodium. Afterwards magnesium stearate was admixed to the previously prepared mixture, and the obtained compression mixture was compressed into tablets.

Preferably, tablets are film coated using a standard water based film coating using HPMC. Example of water based HPMC film coating composition is as follows: HPMC dispersion containing 6wt% hypromellose, 8wt% xylitol, 4.4wt% titanium dioxide, 1.6 % triacetin and 80wt% water.

### I) Different particle size distribution of prasugrel base used in film coated tablets comprising micronized prasugrel base by direct compression, roller compaction and hot melt granulation (Invention)

### I.)/1 Direct compression and roller compaction

With the same composition as stated in table 13 (Example 66) and the same preparation as staded under H.)/1 for direct compression and under H.)/2 for roller compaction.

Particle size distribution of prasugrel base in Examples 66/1, 66/2 and 66/3.
Ex. 66/1: average (mean) particle size 2 µm; d₁₀ <0.7 µm; d₅₀ <1.8 µm; d₉₀ <4.5 µm
Ex. 66/2: average (mean) particle size 5 µm; d₁₀ <1.2 µm; d₅₀ <4.3 µm; d₉₀ <9.9 µm
Ex. 66/3: average (mean) particle size 7 µm; d₁₀ <1.1 µm; d₅₀ <4.2 µm; d₉₀ <10.8 µm; according to laser diffraction method: Pharm. Eur. 2.9.31, Version 6.6., Malvern Mastersizer.

### I.)/2 Hot melt granulation

With the same composition as stated in table 14 and 15 (Example 75b) and the same preparation as staded under H.)/3.

Particle size distribution of prasugrel base in Examples 75b/l
Ex. 75b/l: average (mean) particle size 2 µm; d₁₀ <1.0 µm; d₅₀ <3.6 µm; d₉₀ <8.4 µm; according to laser diffraction method: Pharm. Eur. 2.9.31, Version 6.6., Malvern Mastersizer.

### J) Comparison of stress stability of formulations according to present invention and reference 10 mg tablets (Efient^{®} 10mg batch No.: A594158) (Invention)

Film coated tablets were packaged in aluminum blisters under normal atmosphere or aluminum blisters under nitrogen atmosphere and stored 1 month under the following conditions: 40°C at 75% relative humidity (impurities were determined by HPLC chromatography).

| **Conditions and package** | **Impurities** | **Example 66** | **Example 70** | **Example 74b** | **Reference** |
|---|---|---|---|---|---|
| Untreated t=0 | Max. individual degradation product(%) | **≤0.01** | **0.05** | **≤0.01** | **0.26** |
| | Total impurities (%) | **0.28** | **0.39** | **0.26** | **0.65** |
| stored 1 month at 40°C /75% RH package: Alu blisters -normal atmosphere | Max. individual degradation product(%) | **0.06** | **0.10** | **0.06** | **not analysed** |
| | Total impurities (%) | **0.56** | **0.90** | **0.51** | **not analysed** |
| stored 1 month at 40°C /75% RH package: Alu blisters -nitrogen atmosphere | Max. individual degradation product(%) | **0.09** | **0.20** | **0.09** | **0.41** |
| | Total impurities (%) | **0.46** | **0.70** | **0.51** | **1.09** |

The analytical method used to determine the impurities is characterized by the following features:
Analytical method: Column: ACQUITY UPLC BEH C-18; 50 mm x 2.1 mm i.d., 1.7 µm particle size

### Mobile phase:

Gradient elution:
A: 0.0025M sodium dihidrogen phosphate ; pH-5.5
B: acetonitrile

| | | | | | | | | |
|---|---|---|---|---|---|---|---|---|
| time (min) | 0 | 3 | 18 | 20 | 22 | 25 | 26 | 130 |
| % A | 75 | 65 | 165 | 40 | 20 | 20 | 75 | 175 |
| % B | 25 | 35 | 135 | 60 | 80 | 80 | 25 | 25 |

Flow: 0.45 ml/min
Injection: 2 µl
Detection: UV, 220 nm
Column Temperature: 30°C
Solvent: acetonitrile
Temperature of Sample Solution: 7°C

### K) The content of unbound water of formulations according to present invention

The content of unbound water in film coated tablets was determined in Examples 66, 70 and 74b:

| | **Example 66** | **Example 70** | **Example 74b** |
|---|---|---|---|
| Content of unbound water untreated (t=0) | 1.7 % | 2.3 % | 2.7 % |
| Content of unbound water stored 1 month at 40°C /75% RH package: A1 blisters -normal atmosphere | 1.4 % | 2.0 % | 2.4 % |

Analytical method: sample is being weighed (10-15 mg) in 100 µL aluminium crucible for thermal analysis. The crucible is covered and sealed with piercing lid. The lid is perforated and the sample is inserted into the oven of TG apparatus by the robot. Isothermal temperature program is started and evolved gases from the sample are monitored by the coupled mass spectrometer. At the end of analysis the mass loss due to the water evaporation is being calculated. If needed, the content of residual solvents has to be subtracted from the total mass loss.

Mettler Toledo TGA/DSC 1
Flow rate of nitrogen: 40 ml / min.
Temperature program: isothermal 2 h/ 80 °C, 3h/ 70 °C, - depends on how strongly the crystal water in the sample is bound.

Mass spectrometer Pfeiffer Vacuum Thermostar - monitoring masses from 10-120.

### Example 76: Preparation of prasugrel base

### 2-Acetoxy-5-(a-cyclopropylcarbonyl-2-fluorobenzyl)-4,5,6,7-tetrahydrothieno[3,2-c]pyridine

### (a) Cyclopropyl 2-fluorobenzyl ketone

To a suspension of magnesium powder (7.2 g) in anhydrous diethyl ether or THF (60 ml) was added a solution of 2-fluorobenzylbromide (30 ml) in diethyl ether (30 ml), then the mixture was stirred at room temperature for 1 hour. The reaction mixture was added dropwise to a solution of cyclopropyl cyanide (18.2 ml) in diethyl ether or THF(120 ml) over 100 minutes. After stirring for 30 minutes at room temperature the stirred mixture was heated under reflux for 1 hour. After the reaction, the reaction mixture was partitioned between ethyl acetate and saturated aqueous ammonium chloride solution. The ethyl acetate layer was washed successively with water, saturated aqueous sodium bicarbonate solution, and saturated aqueous sodium chloride solution, then dried over anhydrous sodium sulfate, and then evaporated under reduced pressure.

### (b)5-(alpha-cyclopropylcarbonyl-2-fluorobenzyl)-2-oxo-2,4,5,6,7,7a-hexahydrothieno[3,2-c]pyridine

To a solution of cyclopropyl 2-fluorobenzyl ketone (8.7 g) obtained in example 29(a) in carbon tetrachloride or dichloromethane (80 ml) was added N-bromosuccinimide (9.6 g) or N-chlorosuccinimide (7.20 g) and benzoyl peroxide (0.5 g), then the mixture was heated under reflux for 6 hours. After the reaction, toluene was added to the reaction mixture and the resulting solid was filtered off. The filtrate was concentrated under reduced pressure. The residue was purified by chromatography on a silica gel column using toluene as the eluant to afford alpha -cyclopropylcarbonyl-2-fluorobenzyl bromide (8.5 g) as a yellow oil.

To a solution of alpha-cyclopropylcarbonyl-2-fluorobenzyl bromide (6.0 g) obtained above in dimethylformamide or acetonitrile (20 ml) was added 2-oxo-2,4,5,6,7,7a-hexahydrothieno[3,2-c]pyridine hydrochloride (4.8 g), which was prepared according to the method described in EP 192535 (Japanese Patent Application Publication No. Sho 61-246186) and potassium bicarbonate (7.0 g). Also sodium bicarbonate may be appliled. After stirring the mixture at room temperature for 2 hours the reaction mixture was partitioned between ethyl acetate and water. The ethyl acetate layer was washed with saturated aqueous sodium chloride solution, then dried over anhydrous magnesium sulfate, and evaporated under reduced pressure. After purification of the residue by chromatography on a silica gel column using toluene/ethyl acetate = 3/1 as the eluant, the product was crystallized from diisopropyl ether to afford the desired product

### (c)2-Acetoxy-5-(alpha-cyclopropylcarbonyl-2-fluorobenzyl)-4,5,6,7-tetrahydrothieno[3,2-c]pyridine

To a solution of 5-(alpha -cyclopropylcarbonyl-2-fluorobenzyl)-2-oxo-2,4,5,6,7,7a-hexahydrothieno[3,2-c]pyridine (2.6 g) obtained in reference example 1(b) in a mixture of dimethylformamide (10 ml) and acetic anhydride (5 ml), cooled in an ice bath, was added sodium hydride (60% dispersion in mineral oil, 0.35 g), then the mixture was stirred at the same temperature for 30 minutes, and then at room temperature for 3 hours. After the reaction, the mixture was extracted with ethyl acetate and the extract was washed with saturated aqueous sodium chloride solution, then dried over anhydrous sodium sulfate, and concentrated under reduced pressure. After purification of the residue by chromatography on a silica gel column using toluene/ethyl acetate = 3/1 as the eluant, the product was crystallized from diisopropyl ether to afford the title compound as white crystals, Form I as according to EP 542 411.

### Example 77: Preparation of prasugrel base form II

Prasugrel form I (30 g) was dissolved in 1,4-dioxane (60 ml) by heating to reflux. The mixture was filtered hot and the clear filtrate was cooled first to room temperature within 1 h, then to cca. 10 °C for about 1 - 2 hours. The solid was filtered, washed with *tert*-butyl methyl ether (25 ml) and air-dried. Yield of dry material was 13,54 g.

### Example 78: Preparation of prasugrel hydrochloride

A.) To a solution of 5-[2-cyclopropyl-1-(2-fluorophenyl)-2-oxoethyl]-4,5,6,7-tetrahydrothieno[3,2-c]pyridin-2-yl acetate (450 g) in acetone (4.2 L) was added dropwise concentrated hydrochloric acid (36 %, 100 mL) in 5 minutes with stirring at a temperature of 30 °C. After approx. 30 minutes primary nucleation occurred. The suspension was stirred at 30 °C for the next 90 minutes for the crystallization to complete. The resulting material was separated by filtration and washed with 300 mL of acetone.
   The obtained product was dried in a vacuum oven at 40 °C and 100 mbar for 8 hours (yield 90%, 443 g).
B.) To a solution of 5-[2-cyclopropyl-1-(2-fluorophenyl)-2-oxoethyl]-4,5,6,7-tetrahydrothieno[3,2-c]pyridin-2-yl acetate (5 g) in acetone (50 mL) was added in one portion concentrated hydrochloric acid (36 %, 1,1 mL) with stirring at a temperature of 40 °C. After approx. 30 minutes primary nucleation occurs. The suspension was stirred at 40 °C for the next 90 minutes for the crystallization to complete. The resulting material was separated by filtration and washed with 3.0 mL of acetone.
   The obtained product was dried in a vacuum oven at 40 °C and 100 mbar for 8 hours (yield 85%, 4.64 g).
   Tₒₙₛₑₜ 183.8 °C
   Tₘ = 190.3 5 °C (capillary method)
C.) To a solution of 5-[2-cyclopropyl-1-(2-fluorophenyl)-2-oxoethyl]-4,5,6,7-tetrahydrothieno[3,2-c]pyridin-2-yl acetate (5 g) in acetone (50 mL) was added in one portion concentrated hydrochloric acid (36 %, 1,1 mL) with stirring at a temperature of 20 °C. After approx. 30 minutes primary nucleation occurs. The suspension was stirred at 20 °C for the next 90 minutes for the crystallization to complete. The resulting material was separated by filtration and washed with 3.0 ml of acetone.

The obtained product was dried in a vacuum oven at 40 °C and 100 mbar for 8 hours (yield 89%, 4.86 g).
Tₒₙₛₑₜ 182.0 °C
Tₘ = 188.9 °C (capillary method)

## Claims

1. A process of forming a compressed mixture comprising
a.) an effective amount of prasugrel base or its pharmaceutically acceptable salt,
b.) pharmaceutically acceptable additives suitable for the preparation of solid oral dosage forms, **characterized in that** lactose is not used,
by solvent free technological methods comprising the steps of
i.) optionally grinding the active agent and pharmaceutical additives, mixing them, or mixing them first and grinding them together,
ii.) subjecting a homogenous mixture of the active agent and additives to compression, the average particle size of prasugrel or its pharmaceutically acceptable salt being in the range of 0.2 µm to 150 µm.

2. A process according to claim 1, **characterized in that** the comprimate of step ii.) is further ground, and the obtained granulate is mixed with at least one additional pharmaceutical additive selected from disintegrant, diluent, glidant, lubricant and compressed into tablets.

3. A process according to claim 1, **characterized in that** the process is a direct tabletting process.

4. A process according to claim 1, **characterized in that** the active ingredient and solid additives are subjected to solvent free granulation using at least one additive having melting or glass transition point below 180°C, preferably below 150°C and even more preferably below 100°C by melt extrusion or melt granulation.

5. A process according to any of claims 1 to 4, **characterized in that** unbound water of the composition is controlled to be below 4 weight%, preferably below 3.5 weight% and more preferably below 3 weight% of the composition.

6. A process according to any of claims 1 to 5, **characterized in that** prasugrel base or its pharmaceutically acceptable salt is ground to a particle size of between 0.2 micrometers and 150 micrometers, preferably between 0.2 micrometers and 30 micrometers, most preferably between 1 and 10 micrometers, optionally together with the solubility enhancer.

7. Pharmaceutical composition comprising or consisting of a compressed mixture, said compressed mixture being obtainable according to the process of claim 1.

8. A pharmaceutical composition according to claim 7, **characterized in that** unbound water of the composition is controlled to be below 4 weight%, preferably below 3.5 weight% and more preferably below 3 weight% of the composition.

9. A pharmaceutical composition according to claims 7 or 8, **characterized in that** it comprises a solubility enhancer.

10. A pharmaceutical composition according to claims 7 to 9, **characterized in that** the solubility enhancer is ground together with prasugrel base or its pharmaceutically acceptable salt to a particle size of between 0.2 micrometers and 150 micrometers, preferably between 0.2 micrometers and 30 micrometers, most preferably between 1 and 10 micrometers.

11. A process or composition according to claims 1 to 10, **characterized in that** amorphous prasugrel base is used.

12. A process or composition according to claims 1 to 11, **characterized in that** organic acids are used as solubilizers.

13. A process or composition according to claims 1 to 12, **characterized in that** they comprise an antioxidant agent, preferably that the mixture comprising a prasugrel base or a pharmaceutically acceptable salt thereof and pharmaceutically acceptable additives and/or the pharmaceutical composition comprises an antioxidant agent.

14. A pharmaceutical composition according to claims 7 to 13, **characterized in that** the pharmaceutical composition comprises between 1.0 to 30.0 % by weight of the active ingredient, 0.5 to 5.0 % by weight of a lubricant, 0.0 to 15.0 % by weight of a binder, and the total sum of the combination of components is 100%.

15. A pharmaceutical composition according to claims 7 to 14, **characterized in that** it is coated with a coatings having decreased permeability for gases such as oxygen and/or water vapour based on cellulose ethers such as hyprolose or hypromelose, block copolymer of polyvinyl alcohol and polyethylene glycol (preferably Kollicoat IR or Kollicoat protect), polyvinyl alcohol, aminoalkyl methacrylate copolymers such as Eudragit EPO, methacrylic acid copolymers such as Eudragit L, or salts of carboxymethylcellulose.

16. Pharmaceutical composition according to any of claims 7 to 15, for use in the treatment, alleviation and/or prevention of thrombosis and/or for use as an adjunct to percutaneous coronary intervention procedures.

17. Use of a pharmaceutical composition according to any of claims 7 to 15, for the preparation of a medicament for the treatment, alleviation and/or prevention of thrombosis and/or for the alleviation of percutaneous coronary intervention procedures.

## Patentansprüche

1. Verfahren zum Bilden eines komprimierten Gemisches umfassend
a.) eine wirksame Menge von Prasugrel-Base oder ihres pharmazeutisch verträglichen Salzes,
b.) pharmazeutisch verträgliche Additive, die zur Herstellung von festen oralen Dosierungsformen geeignet sind, **dadurch gekennzeichnet, dass** keine Lactose verwendet wird,
durch lösungsmittelfreie technologische Verfahren umfassend die Schritte von
i.) gegebenenfalls Mahlen des aktiven Mittels und der pharmazeutischen Additive, Mischen von diesen, oder Mischen von diesen zuerst und zusammen Mahlen von diesen,
ii.) Unterwerfen eines homogenen Gemisches des aktiven Mittels und der Additive einer Komprimierung,
wobei die mittlere Teilchengröße von Prasugrel oder seinem pharmazeutisch verträglichen Salz im Bereich von 0,2 µm bis 150 µm liegt.

2. Verfahren gemäß Anspruch 1, **dadurch gekennzeichnet, dass** das Komprimat von Schritt if.) weiter gemahlen wird und das erhaltene Granulat mit mindestens einem zusätzlichen pharmazeutischen Additiv, ausgewählt aus Sprengmittel, Verdünnungsmittel, Fließreguliermittel, Gleitmittel, gemischt wird und zu Tabletten komprimiert wird.

3. Verfahren gemäß Anspruch 1, **dadurch gekennzeichnet, dass** das Verfahren ein direktes Tablettierungsverfahren ist.

4. Verfahren gemäß Anspruch 1, **dadurch gekennzeichnet, dass** der aktive Bestandteil und die festen Additive einer lösungsmittelfreien Granulierung unter Verwendung von mindestens einem Additiv mit einem Schmelz- oder Glasübergangspunkt von niedriger als 180°C, bevorzugt niedriger als 150°C und noch stärker bevorzugt niedriger als 100°C durch Schmelzextrusion oder Schmelzgranulierung unterworfen werden.

5. Verfahren gemäß einem der Ansprüche 1 bis 4, **dadurch gekennzeichnet, dass** nicht gebundenes Wasser der Zusammensetzung so gesteuert wird, dass es weniger als 4 Gew.-%, bevorzugt weniger als 3,5 Gew.-% und stärker bevorzugt weniger als 3 Gew.-% der Zusammensetzung beträgt.

6. Verfahren gemäß einem der Ansprüche 1 bis 5, **dadurch gekennzeichnet, dass** die Prasugrel-Base oder ihr pharmazeutisch verträgliches Salz zu einer Teilchengröße zwischen 0,2 Mikrometern und 150 Mikrometern, bevorzugt zwischen 0,2 Mikrometern und 30 Mikrometern, am stärksten bevorzugt zwischen 1 und 10 Mikrometern, gegebenenfalls zusammen mit dem Löslichkeitsverbesserer, gemahlen wird.

7. Pharmazeutische Zusammensetzung umfassend oder bestehend aus einem komprimierten Gemisch, wobei das komprimierte Gemisch gemäß dem Verfahren nach Anspruch 1 erhalten werden kann.

8. Pharmazeutische Zusammensetzung gemäß Anspruch 7, **dadurch gekennzeichnet, dass** nicht gebundenes Wasser der Zusammensetzung so gesteuert wird, dass es weniger als 4 Gew.-%, bevorzugt weniger als 3,5 Gew.-% und stärker bevorzugt weniger als 3 Gew.-% der Zusammensetzung beträgt.

9. Pharmazeutische Zusammensetzung gemäß den Ansprüchen 7 oder 8, **dadurch gekennzeichnet, dass** sie einen Löslichkeitsverbesserer umfasst.

10. Pharmazeutische Zusammensetzung gemäß den Ansprüchen 7 bis 9, **dadurch gekennzeichnet, dass** der Löslichkeitsverbesserer zusammen mit der Prasugrel-Base oder ihrem pharmazeutisch verträglichen Salz zu einer Teilchengröße zwischen 0,2 Mikrometern und 150 Mikrometern, bevorzugt zwischen 0,2 Mikrometern und 30 Mikrometern, am stärksten bevorzugt zwischen 1 und 10 Mikrometern gemahlen wird.

11. Verfahren oder Zusammensetzung gemäß den Ansprüchen 1 bis 10, **dadurch gekennzeichnet, dass** amorphe Prasugrel-Base verwendet wird.

12. Verfahren oder Zusammensetzung gemäß den Ansprüchen 1 bis 11, **dadurch gekennzeichnet, dass** organische Säuren als Lösungsvermittler verwendet werden.

13. Verfahren oder Zusammensetzung gemäß den Ansprüchen 1 bis 12, **dadurch gekennzeichnet, dass** sie ein Antioxidationsmittel umfassen, bevorzugt dass das Gemisch, umfassend eine Prasugrel-Base oder ein pharmazeutisch verträgliches Salz davon und pharmazeutisch verträgliche Additive, und/oder die pharmazeutische Zusammensetzung ein Antioxidationsmittel umfasst.

14. Pharmazeutische Zusammensetzung gemäß den Ansprüchen 7 bis 13, **dadurch gekennzeichnet, dass** die pharmazeutische Zusammensetzung zwischen 1,0 bis 30,0 Gew.-% des aktiven Bestandteils, 0,5 bis 5,0 Gew.-% eines Gleitmittels, 0,0 bis 15,0 Gew.-% eines Bindemittels umfasst und dass die Gesamtmenge der Kombination der Komponenten 100 % beträgt.

15. Pharmazeutische Zusammensetzung gemäß den Ansprüchen 7 bis 14, **dadurch gekennzeichnet, dass** sie mit einem Überzug/Überzügen mit erniedrigter Permeabilität für Gase wie Sauerstoff und/oder Wasserdampf auf der Basis von Celluloseethern wie Hyprolose oder Hypromelose, Blockcopolymer von Polyvinylalkohol und Polyethylenglycol (bevorzugt Kollicoat IR oder Kollicoat Schutz), Polyvinylalkohol, Aminoalkylmethacrylat-Copolymeren wie Eudragit EPO, Methacrylsäure-Copolymeren wie Eudragit L, oder Salzen von Carboxymethylcellulose überzogen ist.

16. Pharmazeutische Zusammensetzung gemäß einem der Ansprüche 7 bis 15 zur Verwendung bei der Behandlung, Linderung und/oder Vorbeugung von Thrombose und/oder zur Verwendung als ein Hilfsmittel bei perkutanen koronaren Interventionsverfahren.

17. Verwendung einer pharmazeutischen Zusammensetzung gemäß einem der Ansprüche 7 bis 15 zur Herstellung eines Medikaments zur Behandlung, Linderung und/oder Vorbeugung von Thrombose und/oder zur Erleichterung von perkutanen koronaren Interventionsverfahren.

## Revendications

1. Procédé de formation d'un mélange compressé, comprenant
a) une quantité efficace de prasurgrel base ou de son sel pharmaceutiquement acceptable,
b) des additifs pharmaceutiquement acceptables appropriés pour la préparation de formes de dosage oral solides, **caractérisé en ce que** le lactose n'est pas utilisé,
par des procédés technologiques sans solvant, comprenant les étapes de
i) broyage éventuel de l'agent actif et des additifs pharmaceutiques, leur mélange ou leur mélange en premier puis leur broyage,
ii) soumettre à compression un mélange homogène de l'agent actif et des additifs, la taille moyenne des particules de prasugrel ou de son sel pharmaceutiquement acceptable se situant dans la plage allant de 0,2 µm à 150 µm.

2. Procédé selon la revendication 1, **caractérisé en ce que** le comprimé de l'étape ii) est encore broyé, et le granulé obtenu est mélangé avec au moins un additif pharmaceutique supplémentaire parmi les agents de désintégration, les diluants, les agents de coulance, les lubrifiants, et compressé en comprimés.

3. Procédé selon la revendication 1, **caractérisé en ce que** le procédé est un procédé de compression directe.

4. Procédé selon la revendication 1, **caractérisé en ce que** l'ingrédient actif et les additifs solides sont soumis à la granulation sans solvant à l'aide d'au moins un additif ayant un point de fusion ou de transition vitreuse inférieur à 180°C, de préférence inférieure à 150°C et de manière encore plus préférée, inférieure à 100°C par extrusion à l'état fondu ou granulation à l'état fondu.

5. Procédé selon l'une quelconque des revendications 1 à 4, **caractérisé en ce que** l'eau non liée de la composition est contrôlée à moins de 4% en poids, de préférence moins de 3,5% en poids et de manière plus préférée, moins de 3% en poids de la composition.

6. Procédé selon l'une quelconque des revendications 1 à 5, **caractérisé en ce que** le prasugrel base ou son sel pharmaceutiquement acceptable est broyé à une taille de particules allant de 0,2 µm à 150 µm, de préférence de 0,2 µm à 30 µm, de manière la plus préférée de 1 à 10 µm, le cas échéant avec l'agent augmentant la solubilité.

7. Composition pharmaceutique comprenant ou consistant en un mélange comprimé, ledit mélange comprimé pouvant être obtenu selon le procédé de la revendication 1.

8. Composition pharmaceutique selon la revendication 7, **caractérisée en ce que** l'eau non liée de la composition est contrôlée à moins de 4% en poids, de préférence moins de 3,5% en poids et de manière plus préférée, moins de 3% en poids de la composition.

9. Composition pharmaceutique selon la revendication 7 ou 8, **caractérisée en ce qu'**elle comprend un agent augmentant la solubilité.

10. Composition pharmaceutique selon des revendications 7 à 9, **caractérisée en ce que** l'agent augmentant la solubilité est broyé avec le prasugrel base ou son sel pharmaceutiquement acceptable à une taille des particules allant de 0,2 µm à 150 µm, de préférence de 0,2 µm à 30 µm, de manière la plus préférée de 1 à 10 µm.

11. Procédé ou composition selon des revendications 1 à 10, **caractérisé en ce que** le prasugrel base amorphe est utilisé.

12. Procédé ou composition selon des revendications 1 à 11, **caractérisé en ce que** des acides organiques sont utilisés comme solubilisants.

13. Procédé ou composition selon des revendications 1 à 12, **caractérisé en ce qu'**il comprend un agent antioxydant, de préférence que le mélange comprenant un prasugrel base ou un sel pharmaceutiquement acceptable de celui-ci et des additifs pharmaceutiquement acceptables et/ou la composition pharmaceutique comprend un agent antioxydant.

14. Composition pharmaceutique selon des revendications 7 à 13, **caractérisée en ce que** la composition pharmaceutique comprend de 1,0 à 30,0% en poids de l'ingrédient actif, 0,5 à 5,0% en poids d'un lubrifiant, 0,0 à 15,0% en poids d'un liant, et la somme totale de la combinaison de composants fait 100%.

15. Composition pharmaceutique selon des revendications 7 à 14, **caractérisé en ce qu'**elle est revêtue par un revêtement ayant une perméabilité diminuée aux gaz tels que l'oxygène et/ou la vapeur d'eau, à base d'éthers de cellulose comme l'hyprolose ou l'hypromelose, des copolymères séquences de poly(alcool vinylique) et de polyéthylèneglycol (de préférence, Kollicoat IR ou Kollicoat protect), le poly(alcool vinylique), des copolymères de méthacrylate d'aminoalkyle tels que Eudragit EPO, des copolymères d'acide méthacrylique tels que Eudragit L, ou des sels de carboxyméthylcellulose.

16. Composition pharmaceutique selon l'une quelconque des revendications 7 à 15, à utiliser pour traiter, soulager et/ou prévenir la thrombose et/ou à utiliser comme additif pour des procédures percutanées d'intervention coronaire.

17. Utilisation d'une composition pharmaceutique selon l'une quelconque des revendications 7 à 15, pour la préparation d'un médicament pour traiter, soulager et/ou prévenir la thrombose et/ou pour soulager les procédures percutanées d'intervention coronaire.
